# EUROPÄISCHE PATENTSCHRIFT

(11) **EP 0 648 114 B1**
(45) Veröffentlichungstag und Bekanntmachung des Hinweises auf die Patenterteilung: **17.09.1997**
(21) Anmeldenummer: 93913004.3
(22) Anmeldetag: 17.06.1993
(51) Int. Cl.: A61K 9/127, A61K 31/685

(54) **LIPOSOMEN, VERFAHREN ZU IHRER HERSTELLUNG UND IHRE VERWENDUNG ZUR HERSTELLUNG EINES ARZNEIMITTELS**
LIPOSOMES, METHOD OF PREPARING THEM AND THEIR USE IN THE PREPARATION OF DRUGS
LIPOSOMES, PROCEDE CONCERNANT LEUR FABRICATION, ET LEUR UTILISATION DANS LA FABRICATION D'UN MEDICAMENT

(30) Priorität: 08.07.1992 DE 4222447; 25.09.1992 DE 4232231
(43) Veröffentlichungstag der Anmeldung: 19.04.1995
(73) Patentinhaber: DIANORM G. Maierhofer GmbH, 81247 München (DE)
(72) Erfinder: MAIERHOFER, Günther, D-8000 München 65 (DE); HÖFER, Paul, D-8057 Dietersheim (DE); ROTTMANN, Oswald, D-8050 Freising (DE)
(74) Vertreter: Vogelsang-Wenke, Heike
(86) Internationale Anmeldenummer: EP9301545
(87) Internationale Veröffentlichungsnummer: WO9401089

(56) Entgegenhaltungen:
- EP-A- 0 475 160
- DE-A- 4 017 979
- US-A- 4 666 893
- US-A- 4 999 344

## Beschreibung

Die vorliegende Erfindung bezieht sich auf Liposomen ohne darin enthaltenen Wirkstoff, auf die Verwendung der Liposomen zum Herstellen eines Arzneimittels und auf ein Arzneimittel.

Liposomen sind kugelförmige, von mindestens einer völlig geschlossenen Lipid-Doppelschicht umgebene, wäßrige Kompartimente, die einen Durchmesser von 20 nm bis zu mehreren 1000 nm haben können. Wegen ihrer dem Aufbau einer Zellmembran vergleichbaren Membranstruktur dienen sie häufig als Modellsystem für die Untersuchung von Membraneigenschaften in vitro. In den letzten Jahren haben sie zunehmend Bedeutung als Vehikel für den Transport pharmazeutischer und kosmetischer Wirkstoffe gewonnen, da sie aufgrund ihrer Struktur hydrophile Wirkstoffe in das wäßrige Kompartiment einschließen oder hydrophobe Wirkstoffe in die Liposomenmembran integrieren können.

Verfahren zum Herstellen von Liposomen sind im Stand der Technik bereits bekannt. Ebenfalls bekannt sind Verfahren zum Herstellen liposomaler Arzneimittel. So ist in der EP-A-56781 ein Verfahren zur Herstellung liposomaler Arzneimittel beschrieben, demzufolge aus Lipid und einem Tensid gebildete, in wäßriger Phase vorliegender Assoziate durch Entzug des Tensids in Liposomen überführt werden. Die Zugabe von Arzneistoffen oder pharmazeutischen Wirkstoffen vor Entzug des Tensids führt zu Aufnahme dieser Wirkstoffe in die Liposomen. Das molare Verhältnis von Lipid-Doppelschichtbildner und Tensid wird mit 0,1 bis 2 angegeben.

EP-A-220797 beschreibt die Herstellung von Liposomen aus Phospholipid und hydrophilen, nicht-ionischen Tensiden. Bei Anwesenheit von Wirkstoffen während der Liposombildung werden diese in die Liposomen aufgenommen.

EP-A-130577 beschreibt die Herstellung von Liposomen durch Mischen von Lipid und einem wasserlöslichen, nicht-flüchtigen Lösungsmittel und Dispergieren der Lösung in Wasser. Ein pharmazeutischer Wirkstoff kann durch Mischen des organischen Lösungsmittels, das die Membranbestandteile enthält, mit einem die Wirkstoffe enthaltenden wäßrigen Medium in die Liposomen eingeschlossen werden.

EP-A-475160 beschreibt die Herstellung von "Kleinsttröpfchen" zur Applikation von Wirkstoffen.

Den Liposomen aus dem Stand der Technik ist gemeinsam, daß sie alle letztendlich als Träger für pharmazeutische Wirkstoffe, die entweder in das wäßrige Kompartiment des Liposoms eingeschlossen oder in die Membran integriert werden, konzipiert sind.

Aufgabe der vorliegenden Erfindung ist es nunmehr, neuartige Liposomen zur Verfügung zu stellen.

Diese Aufgabe wird erfindungsgemäß durch Liposomen ohne darin enthaltenen Wirkstoff gelöst, die zur Verwendung als Arzneimittel vorgesehen sind und erhältlich sind durch
(a) Mischen von Doppelschicht-bildenden Lipiden, die mindestens einen Anteil von ungesättigten Fettsäureketten enthalten, wobei die Lipide vor dem Mischen entweder als solche vorliegen oder in einem mit Wasser mischbaren Lösungsmittel gelöst sind und in der Mischung in einer Konzentration von 0,625 mMol/l bis 187,5 mMol/l, bevorzugt 37,7 bis 150 mMol/l und besonders bevorzugt 62,5 bis 125,0 mMol/l vorliegen, mit einer wäßrigen Lösung von Gallensäure und/oder mindestens einem ihrer Derivate, wobei das molare Verhältnis von Lipid zu Gallensäure und/oder deren Derivaten (L:G) 3,1 bis 5,5 beträgt, und
(b) Zuführen mechanischer Energie.

Überraschenderweise hat sich erwiesen, daß die erfindungsgemäßen Liposomen, obwohl sie keinen pharmazeutischen oder kosmetischen Wirkstoff enthalten, zur Prophylaxe und Therapie einer Vielzahl von Erkrankungen geeignet sind. Diese vollkommen unerwarteten Eigenschaften der Liposomen, die im folgenden noch näher erläutert werden, sind auf die neue Zusammensetzung der erfindungsgemäßen Liposomen, d.h. ihren Aufbau aus Doppelschicht-bildenden Lipiden mit mindestens einem Anteil von ungesättigten Fettsäureketten in Verbindung mit Gallensäure und/oder Gallensäurederivaten zurückzuführen.

Erfindungsgemäß ist es bevorzugt, daß es sich bei den Lipiden und/oder den Gallensäurederivaten um physiologisch verträgliche Verbindungen, bevorzugt natürlich auftretende Verbindungen handelt. Gallensäure selbst z.B. kommt physiologisch in Form ihrer Konjugate mit Glycin oder Taurin als Gallenbestandteil vor, und ist auch im Blut in einer Konzentration von 1 bis 2 mg pro 100 ml enthalten.

Der Begriff "Lipid", so wie er hier verwendet wird, soll neben klassischen Lipiden mit mindestens einem Anteil von ungesättigten Fettsäureketten Lipoide, d.h., lipidähnliche Substanzen umfassen, z.B. Carotinoide, Amide (z.B. Ceramide) und komplexere Verbindungen, z.B. Glykolipide, beispielsweise Cerebroside und Ganglioside. Der Begriff umfaßt schließlich auch Phospholipide, z.B. Sphingomyeline, Lecithine, Kephaline und Cardiolipine. Bevorzugte Lipide sind Phospholipide, Sphingolipide und Glykolipide. Besonders bevorzugte Verbindungen umfassen Cerebroside und Ceramide, natürliche Phosphocholine, Phosphatidsäuren oder phosphatidylglycerine, sowie gegebenenfalls Lysophospholipide. Bevorzugte Lipide haben eine Phasenübergangstemperatur von weniger als 37°C, besonders bevorzugt von weniger als 25°C.

Die erfindungsgemäßen Liposomen können neben den Doppelschicht-bildenden Lipiden auch Lipide enthalten, die keine Doppelschichtbildner sind. Beispiele dafür sind Cholesterine, Sulfatide, Phosphatidylinosite und Phosphatidylinosit phosphate. Gemäß einer besonders bevorzugten Ausführungsform werden natürliche Lipidpräparate für die Herstellung der Liposomen verwendet. Diese enthalten in Abhängigkeit vom Reinheitsgrad meistens Lipide, die zur Bildung von Doppelschichten unfähig sind.

Die Lipide werden zur Herstellung der Liposomen entweder als solche oder in einem mit Wasser mischbaren Lösungsmittel eingesetzt. Bei diesem mit Wasser mischbaren Lösungsmittel handelt es sich z.B. um mindestens einen Alkohol mit 1 bis 6 Kohlenstoffatomen. In einer bevorzugten Ausführungsform wird Ethanol verwendet.

Die Konzentration der Lipide in der Mischung mit einer wäßrigen Lösung von Gallensäure und/oder mindestens einem ihrer Derivate beträgt 0,625 mMol/l bis 187,5 mMol/l, bevorzugt 37,5 bis 150 mMol/l, und besonders bevorzugt 62,5 bis 125,0 mMol/l.

Der Anteil der ungesättigten Fettsäureketten der für die Herstellung der erfindungsgemäßen Liposomen eingesetzten Doppelschicht-bildenden Lipide beträgt mindestens 4 Gew.-%, wie z.B. bei unreinen Lecithinen, bevorzugt 40 bis 80 Gew.-%, bezogen auf den Gesamtlipidgehalt, wie z.B. bei reinen bis hochreinen Lecithinen. Bevorzugte ungesättigte Fettsäureketten sind Palmitolein-, Öl-, Linol-, Linolen- und Arachidonsäure.

Die Konzentration der Gallensäure und/oder ihrer Derivate hängt von der Konzentration der Lipide ab. Bevorzugt beträgt das molare Verhältnis von Lipid zu Gallensäure und/oder Gallensäurederivat (L:G) 2 bis 20, bevorzugt 2,7 bis 6,7, und besonders bevorzugt 3,1 bis 5,5.

Erfindungsgemäß kann jedes Gallensäurederivat verwendet werden. Bevorzugte Gallensäurederivate sind Natriumcholat, Natriumdesoxycholat, Natriumglykocholat, Natriumtaurocholat, Natriumtaurodesoxycholat, Natriumursocholat und Natriumchenoxycholat. Die zugefügte Gallensäure und/oder deren Derivate führt bzw. führen zu einer Herabsetzung der Oberflächenspannung der Liposomen und damit zu einer erhöhten Fusogenität, die ein Verschmelzen der Liposomen mit Körperzellen erleichtert. Es wird ferner angenommen, daß aufgrund einer Wechselwirkung zwischen Oberflächenspannung und Membranpotential nach Fusion von Liposomen mit Zellen eine vermutlich geringfügige und zeitlich mehr oder weniger begrenzte Membranpotentialänderung eintritt. So beschreiben z.B. Olson et al. (10), daß es wenige Minuten nach einer Behandlung von Rattenleberzellen mit Taurocholat zu einer schwachen Depolarisation des Transmembranpotentials kommt. Weiter könnte es möglich sein, daß die auf ihre räumliche Anordnung angewiesenen Membrankomponenten durch Gallensäure bzw. deren Derivate gewisse räumliche Änderungen erfahren, die sich in einer veränderten Durchlässigkeit der Membran oder einer veränderten Produktionsrate für "second messenger" äußern. Das im Liposom enthaltende Tensid darf jedoch weder zu einer Destabilisierung der Liposomenmembran noch - nach Fusion - der Zellmembran führen. Von allen getesteten Tensiden erfüllen Gallensäure und ihre Derivate diese Forderungen am besten. Zudem wurde beobachtet, daß die so hergestellten Liposomen über lange Zeiträume hinweg besonders stabil waren.

Die erfindungsgemäßen Liposomen brauchen, um für die unten beschriebenen prophylaktischen und therapeutischen Verwendungen geeignet zu sein, außer den bereits erwähnten Liposomenbestandteilen keinerlei pharmazeutisch oder kosmetisch aktiven Wirkstoff zu enthalten. Sie werden in der folgenden Beschreibung daher auch als "physiologische Liposomen" bezeichnet. Erfindungsgemäß ist es jedoch möglich, daß sie einen oder mehrere physiologisch verträgliche Zusätze aus der Provitamine, Vitamine, Mineralstoffe, Öle, Kohlenhydrate, Proteine, Aminosäuren und Reduktionsmittel umfassenden Gruppe enthalten. Diese Zusätze sowie alle weiteren in der Beschreibung erwähnten Zusätze können der die Liposomenbestandteile enthaltenden Mischung entweder zusammen mit dem Lipid oder dem Tensid zugefügt oder aber separat Zugesetzt werden.

In einer bevorzugten Ausführungsform enthalten die erfindungsgemäßen Liposomen mindestens ein Provitamin, z.B. β-Carotin und/oder Vitamin oder Derivate davon, bevorzugt aus der die Vitamine A, B, C und E umfassenden Gruppe. Die genannten Provitamine bzw. Vitamine werden dabei bevorzugt in den folgenden Konzentrationen verwendet: β-Carotin: 0,01 bis 0,15 Gew.-%, Ergosterin: 0,001 bis 0,1 Gew.-%, Vitamin A: 0,05 bis 1 Gew.-%, besonders bevorzugt 0,1 bis 0,3 Gew.-%, Vitamine B1, B2, B6 und Nikotinamid: 0,05 bis 0,5 Gew.-%, besonders bevorzugt 0,1 bis 0,3 Gew.-%, Vitamin B12: 0,0001 bis 0,001 Gew.-%, Folsäure und Biotin: 0,0005 bis 0,002 Gew.-%, Pantothenat: 0,5 bis 2,5 Gew.-%, besonders bevorzugt 0,1 bis 0,3 Gew.-%, Vitamin C: 0,1 bis 30 Gew.-%, besonders bevorzugt 4 bis 12 Gew.-% und Vitamin E: 0,01 bis 0,2 Gew.-%, besonders bevorzugt 0,2 bis 1 Gew.-%.

In dem Fall, in dem die Liposomen einen Mineralstoff enthalten, umfaßt dieser bevorzugt mindestens eines der Elemente Li, Na, K, Mg, Ca oder Fe, bevorzugt in der Salzform als Fluorid, Chlorid, Sulfit, Sulfat oder Phosphat. Die Konzentration der Mineralstoffe beträgt dabei bevorzugt 0,01 Gew.-% bis 4 Gew.-%, besonders bevorzugt 0,1 bis 1 Gew.-%.

Die erfindungsgemäßen Liposomen können in einer weiteren Ausführungsform ein oder mehrere Öle enthalten. Bevorzugt enthalten sie mindestens ein pharmazeutisches Öl pflanzlichen oder tierischen Ursprungs; besonders bevorzugte Öle werden dabei aus der Jojobaöl, Borretschöl, Nachtkerzenöl, Kaminöl, Teebaumöl oder Fischöl umfassenden Gruppe ausgewählt. Die Konzentration der Öle beträgt bevorzugt 0,01 Gew.-% bis 4 Gew.-%, besonders bevorzugt 0,1 Gew.-% bis 2 Gew.-%.

Bevorzugte Kohlenhydrate sind Ribose, Arabinose, Glucose, Mannose, Galaktose, Fructose, Sorbose, Saccharose, Lactose, Maltose und gegebenenfalls auch Mucopolysaccharide. Diese Kohlenhydrate können z.B. in einer Konzentration von 0,05 bis 2 Gew.-% verwendet werden.

Bevorzugte Aminosäuren sind essentielle Aminosäuren, also Isoleucin, Leucin, Lysin, Methionin, Phenylalanin, Threonin, Tryptophan und Valin, außerdem Arginin, Histidin, Cystein und Tyrosin. Diese Aminosäuren sowie auch Zitronen- und/oder Brenztraubensäure werden bevorzugt in einer Konzentration von 0,001 bis 0,2 Gew.-% verwendet.

In einer bevorzugten Ausführungsform wird den erfindungsgemäßen Liposomen mindestens ein Antioxidans, bevorzugt mindestens ein physiologisch verträgliches Antioxidans zugefügt. Bei den Antioxidantien handelt es sich bevorzugt um BHA, BHT, Octyl- und Dodecylgallat, SO₂, z.B. in Form von Natriumsulfiten oder Natriumthiosulfit, Milch-, Zitronen-, Weinsäure und/oder deren Salze, Vitamin C, Vitamin E und Harnsäure und deren Salze. Besonders bevorzugte physiologisch verträgliche Antioxidantien sind α-Tocopherol, Bilirubin, Vitamin C, Vitamin E, Harnsäure und deren Salze und/oder Derivate davon. Harnsäure und ihre Salze werden bevorzugt in einer Konzentration von 0,01 bis 1 Gew.-% zugefügt, Vitamin C in einer Konzentration von 0,01 bis 2 Gew.-% und Vitamin E in einer Konzentration von 0,01 bis 0,1 Gew.-%.

Bei Bedarf können die erfindungsgemäßen Liposomen einen oder mehrere übliche Hilfs- und/oder Zusatzstoffe, vorzugsweise Gelbildner, Puffersubstanzen, Membranstabilisatoren und Konservierungsmittel, enthalten.

Die Gelbildner dienen dabei dem Andicken von Formulierungen; dafür können Andickungsmittel, wie z.B. Kollagen und/oder Hyaluronsäure, in einer Konzentration von bevorzugt 0,01 bis 0,2 Gew.-% zugesetzt werden, ebenso organische Hydrogelbildner, z.B. Methyl-, Hydroxyethyl-, Carboxymethylzellulose, Stärke oder Alginat, die in einer Konzentration von 1 bis 6 Gew.-% filmbildend oder in der bevorzugt verwendeten Konzentration von 0,01 bis 2 Gew.-%, besonders bevorzugt 0,01 bis 0,5 Gew.-%, tiefenwirksam sein können, wie z.B. Polyacrylate (Carbopol®, Hostacerin®, Rhodigel®, Hispagel®, Xanthan Gum etc.).

Die Puffersubstanzen werden zugesetzt, um einen gewünschten pH-Wert über längere Lagerperioden zu garantieren. Ihre absolute Konzentration ergibt sich daher immer aus dem gewünschten pH-Wert. Üblicherweise werden z.B. Citrat-, Acetat-, Phosphat- und/oder Zitronensäurepuffer verwendet oder eine der bekannten Pufferlösungen für biologische Medien, z.B. Trispuffer oder Trismaleatpuffer.

Membranstabilisierend können zum einen aus anderen Gründen zugesetzte Substanzen wirken, bei denen die Membranstabilisierung eine Nebenwirkung ist, wie z.B. Mineralstoffe in Salzform, Puffersubstanzen, Antioxidantien und Gallensäure und ihre Derivate. Daneben kann auch Cholesterin in einer Konzentration von 0,1 bis 1 % zugesetzt werden, ebenso Glycerin, Glykol oder Polyethylenglykol in einer Konzentration von 0,1 bis 10 %, bevorzugt 1 bis 5 %.

Als Konservierungsmittel können Ameisensäure in einer Konzentration von bevorzugt 0,03 bis 0,4 Gew.-%, Essigsäure (bevorzugt 0,3 bis 3 Gew.-%), Propion-, Milch- und Sorbinsäure in einer Konzentration von bevorzugt 0,05 bis 6 Gew.-%, besonders bevorzugt 0,05 bis 12 Gew.-%, SO₂ (bevorzugt 0,01 bis 0,6 Gew.-%), Salicylsäure und ihre Salze (bevorzugt 0,01 bis 0,5 Gew.-%), PHB-Ester (bevorzugt 0,05 bis 0,6 Gew.-%), Imidazolidinylharnstoffderivate (bevorzugt 0,01 bis 0,6 Gew.-%), Chlorhexidin, Nipe-Ester® oder Antibiotika zugesetzt werden.

Die erfindungsgemäßen Liposomen werden aus der Mischung der Doppelschicht-bildenden Lipide mit mindestens einem Anteil von ungesättigten Fettsäureketten und einer wäßrigen Lösung von Gallensäure und/oder mindestens einem ihrer Derivate durch Zuführen mechanischer Energie erhalten. Dies kann z.B. durch Rühren, Schütteln, Homogenisieren oder durch andersartige Einwirkung von Scherkräften, z.B. durch Filtrieren, erfolgen. In einer bevorzugten Ausführungsform wird die heterogene Mischung aus Lipid und Gallensäure bzw. Gallensäurederivat einmal bei geringem Überdruck, bevorzugt 10⁵ Pa bis 6 x 10⁵ Pa, mit einem Filter einer Porengröße von 0,1 bis 0,8 µm, bevorzugt 0,15 bis 0,3 µm, und besonders bevorzugt 0,15 bis 0,22 µm, filtriert. Für sterile Liposomenpräparationen beträgt die obere Grenze des Porendurchmessers zweckmäßigerweise 0,22 µm. Wahlweise kann die mechanische Energie z.B. mit einem Rührwerk zugefügt werden. Eine Lagerung des heterogenen Gemisches ohne Störung des Systems durch Zufuhr mechanischer Energie führt nach mehreren Monaten zu einer Selbstbildung von Liposomen.

Die erfindungsgemäßen Liposomen sind zur Verwendung als Arzneimittel geeignet. Sie können dabei auf jede in der Medizin übliche Weise appliziert werden, z.B. dermal, intravenös, oral, subcutan, intramuskulär oder intraperitoneal. Die erfindungsgemäß hergestellte Liposomenpräparation wird dabei im Verhältnis 1:1 bis 1:1000, bevorzugt 1:1 bis 1:20, mit physiologisch verträglichen Verdünnungsmittel verdünnt. Die Wirkung tritt innerhalb kurzer Zeit ein und zeigt sich im Fall einer Therapie durch rasches Abklingen der klinischen Symptome, wie unten gezeigt wird.

Weiter wird ein Verfahren zum Herstellen von Liposomen zur Verfügung gestellt, bei dem die Doppelschicht-bildenden Lipide, die mindestens einen Anteil von ungesättigten Fettsäureketten enthalten, mit einer wäßrigen Lösung von Gallensäure und/oder mindestens einem ihrer Derivate gemischt werden, wobei die Lipide vor dem Mischen entweder als solche vorliegen, oder in einem mit Wasser mischbaren Lösungsmittel gelöst sind, und der Mischung anschließend mechanische Energie zugeführt wird.

Die mechanische Energie kann durch Schütteln, Rühren, Homogenisieren oder einmaliges Filtrieren zugefügt werden. Die Filtration erfolgt dabei bevorzugt bei geringem Überdruck, insbesondere 10⁵ Pa bis 6 x 10⁵ Pa; die Porengröße der Filter beträgt dabei 0,1 bis 0,8 µm, bevorzugt 0,15 bis 0,3 µm und besonders bevorzugt 0,15 bis 0,22 µm.

Die Mischung aus den Doppelschicht-bildenden Lipiden und der wäßrigen Lösung von Gallensäure und/oder einem ihrer Derivate wird bei 0 bis 95°C hergestellt, bevorzugt bei 18 bis 70°C, und besonders bevorzugt bei 18 bis 38°C.

Bei der Herstellung der Mischung sollte ein pH von 4,0 bis 10,0, bevorzugt 5,5 bis 7,5, eingehalten werden.

Die erfindungsgemäß mittels Filtration durch Filter mit einer Porengröße von 0,22 bis 0,45 µm hergestellten Liposomen weisen einen Durchmesser von ungefähr 50 bis ungefähr 200 nm auf, wobei der mittlere Durchmesser ungefähr 80 bis ungefähr 120 nm beträgt. Die Durchmesser wurden mittels Elektronenmikroskopie und Photokorrelationspektroskopie bestimmt.

Die erfindungsgemäßen Liposomen werden bevorzugt zur Herstellung eines Arzneimittels verwendet. Überraschenderweise wurde festgestellt, daß die erfindungsgemäßen Liposomen gegen eine Gruppe von sehr unterschiedlichen Erkrankungen, z.B. Allergien, virale Infektionen, Entzündungen und eine bestimmte Art von Schmerzen, wirkungsvoll sind. All diesen Erkrankungen ist jedoch gemeinsam, daß sie ihren Ursprung in einer bestimmten "Labilität", bzw. "Akzeptanz" der Plasmamembran der hiervon betroffenen Zellen haben. So werden bestimmte allergische Reaktionen durch die Destabilisierung der Mastzellmembran ausgelöst, Nervenimpulse durch destabilisierte Zellmembranen generiert, und Viren können nur in diejenigen Zellen eindringen, deren Zellmembran zum Zeitpunkt der Infektion über die nötige Akzeptanz verfügt (Einzelheiten werden in den nachfolgenden Abschnitten I bis V beschrieben).

Die erfindungsgemäßen Liposomen verfügen über antivirale (virustatische), antiallergische, antiphlogistische, analgetische und regenerative bzw. gewebeschützende Eigenschaften. Eine Grundwirkung ist dabei die Beeinflussung der Zellmembran durch die Liposomen nach Interaktion (Fusion) mit Zellen, hierdurch wird möglicherweise zum einen die Mediatorfreisetzung (Exozytose) unterdrückt und zum anderen die Fusogenität der Zellen mit Viruspartikeln herabgesetzt. Vor allem Antioxidantien enthaltende Liposomen dürften für die Neutralisation hochreaktiver Sauerstoffradikale im extra- und intrazellulären Raum verantwortlich sein. Bei Nozirezeptoren tritt möglicherweise nach der Fusion eine Erhöhung der Reizschwelle bzw. Erniedrigung der Aktionspotentials auf und/oder eine Beeinflussung von Rezeptoren (z.B. der Opioidrezeptoren) bzw. entsprechend von assoziierten Membranproteinen. Falls die erfindungsgemäßen Liposomen eine Wirkung auf Membranproteine ausüben, welche die intrazelluläre Menge an "second messenger", wie beispielsweise cAMP, beeinflussen, so können auch bestimmte Peptid- und Proteohormon-korrelierte Erkrankungen (z.B. Diabetes) oder Wachstumsfaktoren-abhängige Zellentartungen (z.B. Tumorentstehung und -wachstum) therapiert werden.

Die erfindungsgemäßen Liposomen sind in der Lage, in das Körpergewebe einzudringen, z.B. in die Haut. Sie interagieren dort - vermutlich durch Fusion - mit Zellen und ändern weniger chemisch als vielmehr physikalisch die Plasmamembran, da die Liposomenmembran aus Komponenten besteht, die auch in der Plasmamembran vorkommen. Hinweise dafür ergeben sich aus dem unter "Antiviraler Behandlung" (nachfolgender Abschnitt I) beschriebenen Experiment, wonach eine Vorinkubation der Zielzellen (Vero) mit den Liposomen die Akzeptanz dieser Zellen für eine nachfolgende Herpesvirus-Infektion deutlich (auf weniger als ein Drittel) herabsetzt. Eventuell wird nach Fusion von Liposomen mit Zellen auch die Fähigkeit einer Zelle zur Exozytose von Vesikeln und damit zur Freisetzung von Mediatoren herabgesetzt. Erst nach einer gewissen Zeit, wenn die durch die Liposomenfusion in die Zellmembran eingebrachten Stoffe (Lipide und Gallensäure und/oder deren Derivate) wieder durch physiologische Vorgänge herausgelöst wurden, erhält die Zelle ihre Fähigkeit zur ursprünglichen Exo- und Endozytose zurück.

Diese physikalischen Auswirkungen auf die Zellmembran können auch therapeutische Ansätze auf pathologisch veränderte Körperzellen liefern, bei denen Veränderungen der Zelloberfläche für das krankhafte Geschehen mit verantwortlich sind. So etwa sind die Fähigkeiten von Tumorzellen zur aktiven Lokomotion, homo- und heterotypischen Aggregation und die verschiedenen Erkennungs- und Adhaesionsmechanismen Ausdruck einer vom Normalzustand abweichenden Zellmembran.

Grundsätzlich kann davon ausgegangen werden, daß physiologische Liposomen, gegebenenfalls einschließlich der genannten Zusatzstoffe, zur Behandlung all derjenigen Symptome herangezogen werden können, bei denen (wie zuvor beschrieben) eine gewisse "Labilität" oder "Akzeptanz" der Zellmembranen als zumindest eines der verursachenden Prinzipien der Krankheit auftritt. Da dies nicht immer offensichtlich ist, wurden fünf Symptomkomplexe erläutert (siehe unten, Abschnitte I bis V). Zudem finden sich für einzelne Erkrankungen spezielle zusätzliche Therapiemechanismen der Liposomen, auf die in den einzelnen Abschnitten näher eingegangen werden wird.

Die erfindungsgemäßen Liposomen werden insbesondere zur Prophylaxe und/oder zur Therapie von Erkrankungen, die von Viren mit Lipidhülle, insbesondere Viren aus der die Familien Herpesviridae, Orthomyxoviridae, Retroviridae und Hepadnaviridae umfasssenden Gruppe hervorgerufen werden.

Eine weitere bevorzugte Verwendung ist die zur Prophylaxe und/oder Therapie von Erkrankungen, die von Viren ohne Lipidhülle, insbesondere Viren aus der die Familien Adenoviridae, Papovaviridae und Picornaviridae umfassenden Gruppe, hervorgerufen werden.

Die erfindungsgemäßen Liposomen eignen sich ferner zur Therapie von durch Mycoplasmataceae, Chlamydiaceae und Rickettsiaceae hervorgerufenen Erkrankungen.

Ein hauptsächliches Verwendungsgebiet der erfindungsgemäßen Liposomen ist die Prophylaxe und/oder Therapie allergischer Erkrankungen, insbesondere der Haut und der Schleimhaut. Die erfindungsgemäßen Liposomen eignen sich dabei zur Verwendung für die Herstellung von Medikamenten zur Prophylaxe und/oder Therapie von Kontakt-, Nahrungsmittel- und Arzneimittelallergien. Weitere Verwendungsmöglichkeiten bestehen in der Herstellung von Arzneimitteln zur Prophylaxe und/oder Therapie von Allergien des atopischen Formenkreises, insbesondere bei Neurodermitis. Im Fall der Verwendung zur Herstellung eines Arzneimittels zur Therapie der Neurodermitis kann dem Medikament weiterhin ein Lokalanaesthetikum in den für Hautschutzmittel zulässigen Konzentrationen zugefügt werden. Bevorzugte Lokalanaesthetika sind dabei Lidocain oder Tetracain.

Die überragenden Eigenschaften der erfindungsgemäßen Liposomen ermöglichen weiter ihre Verwendung zur Herstellung eines Hautschutzmittels, das zur Verwendung im gewerblichen Hautschutz geeignet ist. Eine weitere Verwendungsmöglichkeit besteht in der Verwendung zur Herstellung eines Mittels zur Prophylaxe und/oder Therapie der trockenen Haut, wobei diesem Mittel gegebenenfalls Harnstoff zugesetzt wird.

Ein von den vorgenannten Verwendungsmöglichkeiten vollkommen unabhängiges Gebiet ist die Verwendung der erfindungsgemäßen Liposomen zur Herstellung eines Medikaments zur Prophylaxe und/oder zur Therapie von Schmerzen. Es hat sich gezeigt, daß die erfindungsgemäße Verwendung insbesondere zur Prophylaxe und/oder Therapie des tonischen Schmerzes sowie zur Prophylaxe und/oder zur Therapie von schmerzhaften Muskelverspannungen, Operationsnarben und Phantomschmerzen geeignet ist.

Eine weitere bevorzugte Ausführungsform sieht die Verwendung der erfindungsgemäßen Liposomen zur Herstellung eines Arzneimittels zur Therapie des Weichteilrheumatismus vor.

Es hat sich außerdem gezeigt, daß ein die erfindungsgemäßen Liposomen enthaltendes Arzneimittel zur Behandlung der Zahnhalsempfindlichkeit gut geeignet ist. Eine weitere Verwendungsmöglichkeit ist die Verwendung der erfindungsgemäßen Liposomen zur Prophylaxe und/oder Therapie allergisch und/oder viral bedingter Entzündungen des Auges. Am Auge können die erfindungsgemäßen Liposomen weiterhin zur Prophylaxe und/oder Therapie der Symptomatik des trockenen Auges verwendet werden.

Ganz allgemein eignen sich die erfindungsgemäßen Liposomen weiter zur Herstellung eines Arzneimittels zur Prophylaxe und/oder Therapie entzündlicher Erkrankungen.

Gemäß einer weiteren Ausführungsform der vorliegenden Erfindung werden die erfindungsgemäßen Liposomen zur Herstellung eines Arzneimittels zur Prophylaxe und/oder Therapie von Schädigungen verwendet, die durch natürliches oder synthetisches UV-Licht, durch Strahlung aus radioaktivem Zerfall, durch Röntgenstrahlung oder Hitze verursacht werden.

Es hat sich ferner gezeigt, daß die erfindungsgemäßen Liposomen zur Therapie der rheumatoiden Arthritis geeignet sind.

Erfindungsgemäß können die Liposomen weiter zur Herstellung von Arzneimitteln zur Prophylaxe und/oder Therapie altersbedingter Beschwerden sowie als Radikalfänger und/oder zur Soforthilfe bei verschiedenen Schockzuständen verwendet werden.

Wie oben ausführlich erläutert, beeinflussen die erfindungsgemäßen Liposomen das Membranpotential von mit ihnen fusionierenden Zellen. Daher sind die erfindungsgemäßen Liposomen zur Behandlung von Erkrankungen, die ganz oder teilweise durch vom Normalzustand abweichenden Membranpotentiale der betreffenden Zellen verursacht werden, äußerst gut geeignet.

Durch die Fusion der erfindungsgemäßen Liposomen werden nicht nur ihre Membranbestandteile, sondern auch das wäßrige Kompartiment in die Zelle eingebracht. Die Liposomen eignen sich daher auch zur Herstellung von Arzneimitteln zur Prophylaxe oder Therapie von Erkrankungen, die ganz oder teilweise durch einen vom Normalzustand abweichenden Flüssigkeitsverlust der betroffenen Zellen verursacht werden.

Ebenfalls bereits erwähnt wurde, daß die Oberflächenspannung von Zellen, die mit den erfindungsgemäßen Liposomen fusionieren, verändert wird. Die erfindungsgemäßen Liposomen eignen sich daher ebenfalls zur Herstellung von Arzneimitteln, die der Prophylaxe und/oder Therapie von Erkrankungen dienen, die ganz oder teilweise durch eine im Verhältnis zum Normalzustand erhöhte Oberflächenspannung der betreffenden Zellmembranen verursacht werden.

Eine weitere Verwendungsmöglichkeit der erfindungsgemäßen Liposomen besteht in der Herstellung von Arzneimitteln zur Prophylaxe und/oder Therapie von Erkrankungen, die durch Schädigungen aufgrund oxidativer Prozesse an zellulären und/oder extrazellulären Strukturen hervorgerufen werden.

Wie oben bereits mehrfach erwähnt worden ist, können die durch Fusion mit den erfindungsgemäßen Liposomen veranderten Membraneigenschaften Einfluß auf die Konzentration der zellulären "second messenger" haben. Die erfindungsgemäßen Liposomen eignen sich daher auch zur Prophylaxe und/oder Therapie von Erkrankungen, die ganz oder teilweise durch vom Normalzustand abweichende intrazelluläre Konzentration eines oder mehrerer "second messenger" verursacht werden.

Beansprucht wird schließlich ein Arzneimittel, das die erfindungsgemäßen Liposomen enthält.

In den nachfolgenden Abschnitten I bis V werden verschiedene Möglichkeiten der Verwendung der erfindungsgemäßen Liposomen beschrieben. In den Fällen, in denen Hypothesen aufgestellt werden können, warum die erfindungsgemäßen Liposomen derart vielfältige und unerwartete Wirkungen haben, werden mögliche Erklärungen diskutiert.

### I. Verwendung der erfindungsgemäßen physiologischen Liposomen zur Prophylaxe und Therapie viraler Infektionen

Die vorliegende Erfindung bezieht sich auf die prophylaktische und therapeutische Anwendung der erfindungsgemäßen Liposomen zur Behandlung viraler Infektionen in vivo und in vitro. Die erfindungsgemäßen Liposomen sind gegen völlig unterschiedliche Virusarten wirksam. In den getesteten effektiven Konzentrationen konnten keinerlei negative Einflüsse der Liposomen auf Zellen bzw. Organismen festgestellt werden.

Die Wirkungsprinzipien aller bisherigen antiviralen Wirkstoffe (außer aktiver und passiver Immunisierung, sowie Unterstützung der Immunabwehr durch körpereigene Substanzen, wie Interleukine und Interferone, sowie Amantadin und Rimantadin, deren Wirkungsweise man noch nicht genau kennt) beruht auf einer Inhibition virusspezifischer Enzyme (Polymerasen), z.B. der Inhibtion der viralen DNA- bzw. RNA-Synthese durch Nukleosidanaloga (9). Da virale Enzyme in Art und Wirkung körpereigenen Enzymen ähneln, sind alle Enzyminhibitoren ab einer gewissen Dosis auch für Zellen bzw. den Wirtsorganismus toxisch. Dies gilt auch für eine Therapie mit Nukleosidanaloga. Hier wird nicht nur die schnelle Replikation der DNA- bzw. RNA-Stränge der Viren inhibiert, sondern auch die körpereigener Erbsubstanzmoleküle, die sich während der Therapie replizieren. Hier treten bei systemischer Applikation die ersten Schädigungen vor allem im Knochenmark, Darmepithel und in den Haarwurzelzellen auf. Besonders kritisch müssen hier die zu erwartenden Langzeitschäden in Form mutagener Veränderungen gesehen werden. Die Therapie ist demnach als eine Gratwanderung zwischen möglichst effektiver, antiviraler Wirkung und möglichst geringer Intoxikation des Betroffenen zu werten. Letztendlich gab es bis zur vorliegenden Erfindung keine antivirale Substanz, die ohne größere toxische Nebenwirkungen für den Betroffenen bleibt. Anders als bei der Antibiotikabehandlung bakterieller Infektionen werden virale Infektionen bei Mensch und Tier praktisch ausschließlich durch das körpereigene Immunsystem bekämpft, dessen Ausfall bei Immundefizienz die Hilflosigkeit gegenwärtiger Therapien vor Augen führt (z.B. AIDS).

Überraschenderweise hat sich gezeigt, daß erfindungsgemäße physiologische Liposomen (d. h. Liposomen ohne eigentliches antivirales Therapeutikum) die Ausbreitung von Viren verhindern können. Der Effekt könnte vielleicht auf eine kompetetive Inhibition der Bindung von Viren an die Membran durch physiologische Liposomen zurückgeführt werden. Die möglicherweise durch Rezeptoren bedingte höhere Affinität der Viren zu ihren Wirtszellen kann bei der "Liposomentherapie" durch die weitaus größere Zahl liposomaler Reaktionspartner kompensiert werden. Die in der vorliegenden Erfindung getestete höchste Liposomenkonzentration beträgt durchschnittlich 4,4 x 10¹⁴ Liposomen pro ml. Demgegenüber steht als höchste Herpes simplex-Konzentration in vitro 10⁸ Viren bzw. PFU (plaque forming units) pro ml. Welche Viruskonzentrationen in vivo, z. B. in einer virusbedingten Gewebeläsion, vorliegen, ist nicht bekannt. Es wird jedoch davon ausgegangen, daß wesentlich weniger infektiöse Herpesviren pro ml Gewebeflüssigkeit auftreten. Die Affinität der Viren zur Wirtszelle müßte daher um viele Zehnerpotenzen größer sein als gegenüber den gleichzeitig angebotenen Liposomen, falls die Viren durch die Liposomen nur "behindert" und nicht zerstört werden (letzteres trifft möglicherweise für einen Teil der Viruspopulation zu, siehe nachfolgende Ergebnisse). Die anschließend beschriebenen Resultate zeigen, daß die enorm hohe Zahl applizierbarer Liposomen tatsächlich einen virustatischen Effekt hervorruft, möglicherweise aufgrund "mechanischer" Inhibition.

Eine vorläufige Erklärung für die beobachteten starken antiviralen Eigenschaften physiologischer Liposomen könnte, wie folgt, lauten: Liposomen der verwendeten Art dringen in Organe, Gewebe und Körperflüssigkeiten ein, fusionieren dort mit Viren bzw. Zellen und/oder "besetzen" interzelluläre "Freiräume". Die Wirkungen, die sie entfalten, sind vermutlich vielgestaltig und könnten sich aus einer oder mehreren der nachfolgenden Ursachen ergeben, möglicherweise mit unterschiedlicher Gewichtung je nach Gewebe- bzw. Zelltyp und in Abhängigkeit vom jeweiligen Virus:
1. Die Plasmamembran tierischer Zellen kann innerhalb einer bestimmten Zeit (je nach Zelltyp) nur mit einer limitierten Menge an Partikeln bzw. Vesikeln fusionieren. Die der Endozytose von Viren vorausgehende Bindung erfolgt über sog. "coated pits", mit bestimmten Proteinen ausgekleideten Mulden der Plasmamembran, die etwa zwei Prozent der Oberfläche einer typischen tierischen Zelle ausmachen. Vielleicht "stören" die durch Zellmembran-Liposomen-Fusion eingebrachten Tenside die räumliche Struktur derjenigen Zellmembranbestandteile, welche die "coated pits" bilden, und verhindern so zumindest für einige Zeit die Anlagerung von Viren. Hat eine Zelle bereits eine genügende Anzahl von Liposomen aufgenommen, so ist sie für einige Zeit "inert" gegenüber dem Eindringen von Viren.
2. Der Einbau liposomaler Bestandteile (z. B. Phospholipid und/oder Cholat) in die Plasmamembran könnte zu geringfügigen Membranveränderungen (z. B. Membranpotential, siehe Abschnitt III) führen, die eine virale Exozytose erschweren oder auch die exozytierte virale Hüllstruktur destabilisieren.
3. Die Durchmesser der verwendeten Liposomen liegen im Größenbereich der meisten Viren, nämlich zwischen 20 und 300 nm. Liposomen gleichen - teilweise auch in ihren physikochemischen Oberflächeneigenschaften - demzufolge vielen Viren. Man könnte Liposomen auch als eine Art "Pseudovirus" in dem Sinne bezeichnen, daß sie aufgrund ihrer Größe und physiko-chemischen Eigenschaften über einen ähnlichen Ausbreitungsmodus verfügen, wie ihn viele echte Viren verwenden. Liposomen, die leicht in sehr großen Zahlen beispielsweise in Hautgewebe eindringen, täuschen dort das Vorhandensein von Viren vor, behindern möglicherweise auch virale Bindungsstellen und erlauben den echten Viren dadurch keine weitere Verbreitung. Die durch derartige liposomale "Pseudoviren" hervorgerufene antivirale Wirkung würde sich demzufolge durch eine einfache mechanische Inhibierung bzw. Verdrängungsreaktion manifestieren.
4. Durch die Wechselwirkung von Liposomen vor allem mit zweiwertigen Ionen verändern diese die physikalischen Bedingungen im interzellulären Raum. Möglicherweise kommt es dabei zu einer Verschiebung des Ionengleichgewichtes derart, daß Viren in ihrer Verbreitung gehindert werden. Dies könnte besonders für die von einigen Viren benötigten erhöhten Calciumwerte zutreffen.
5. Eine größere antivirale Wirkung ergibt sich ferner durch Zugabe reduzierender Substanzen, wie z. B. Ascorbinsäure und/oder Sulfit als Einzelsubstanz oder im Gemisch. Vielleicht führt die liposomale Einbringung derartiger Substanzen in das Gewebe dazu, daß durch Virusvermehrung freigesetzte oxidierende Noxen (Radikale aus lysierten Zellen) rechtzeitig abgefangen und so gesunde Nachbarzellen weniger geschädigt werden. Ferner könnten Liposomen, deren Bestandteile durch entsprechende Zusätze in reduzierter Form gehalten werden, allgemein bessere physiologische Eigenschaften aufweisen.
6. Einige Virusfamilien, wie z. B. die Herpesviridae und die Rhinoviridae, zeichnen sich durch eine Labilität gegenüber pH-Werten zwischen 5 und 6 aus. Die verstärkte antivirale Wirkung Ascorbinsäure-enthaltender bzw. anderer "saurer" Liposomen könnte hieraus erklärt werden.
7. Liposomen können mit Viren interagieren, möglicherweise fusionieren und letztendlich Viren neutralisieren. Hierfür ist - den Resultaten aus Beispiel 1 folgend - eine gewisse Liposomenzahl nötig, um Viren zu dieser Interaktion zu zwingen. Die effektive Dichte ergibt sich hauptsächlich aus der Größe der Viren und der Liposomen. Die Neutralisierung selbst könnte bei umhüllten Viren einfach durch Überschreiten einer für das jeweilige Virus wichtigen Grenzgröße (jedes Virus hat eine bestimmte Maximalgröße) bzw. bei nackten Viren durch Umhüllung mit der Liposomenmembran erfolgen. Vermutlich werden die Viren jedoch durch die nach Fusion mit den Liposomen übertragenen Tenside destabilisiert.
8. Liposomen bringen in durch Viren hervorgerufene Gewebeläsionen verwertbares Membranmaterial (Lecithin) ein, was die Regeneration zerstörter Areale beschleunigen könnte.

Aus bisherigen Untersuchungen an Probanden resultiert eine eindeutige antivirale Wirkung erfindungsgemäßer Liposomen gegen Vertreter der Alphaherpesviren, wie Herpes simplex und Varizella zoster, und Betaherpesviren, wie Cytomegalievirus. Die viralen Efflorenzen bei Herpes simplex gehen innerhalb eines Tages deutlich zurück. Die bei manchen Patienten beobachtbaren Schmerzen während einer akuten Phase der Infektion werden durch die Liposomen stark reduziert bzw. verschwinden gänzlich. In einem Fall erwiesen sich Liposomen als wirksam gegenüber einer Aciclovir-resistenten Herpes simplex-Infektion (Heilversuchsstudie am Rambla Hospital, Barcelona). Im Tierversuch konnte eine therapeutische Wirkung bei der Behandlung der infektiösen bovinen Rhinotracheitis erzielt werden, einer durch das bovine Herpesvirus (Typ 1) hervorgerufenen Erkrankung bei Rindern.

In vitro-Versuche mit Herpes simplex Typ 1 (HS 1/5886) an Affennierenzellen (Vero-Zellen) zeigen je nach Bedingung eine über 99 %ige Reduktion der PFU (plaque forming units) nach einmaliger Applikation physiologischer Liposomen. Aufgrund der Resultate aus verschiedenen Testansätzen zeigt sich, daß Liposomen mit Viren interagieren und diese unschädlich machen; ihre Wirkung wird durch Membranveränderungen der Wirtszellen zusätzlich verstärkt. Demzufolge bzw. zumindest in diesem Testsystem dürfte die antivirale Wirkung physiologischer Liposomen hauptsächlich auf Effekten beruhen, wie sie zuvor unter 1. und 7. als mögliche Erklärung beschrieben wurden. Diese Schlußfolgerung gilt nicht zwingend für die Anwendung in vivo, d. h. für das Gewebe oder den ganzen Organismus. In vitro-Tests mit HIV-1 (Retroviridae) und Influenzaviren (Orthomyxoviridae) lassen ebenfalls eine eindeutige Virusreduktion nach Zugabe von Liposomen erkennen.

Sollten die antiviralen Eigenschaften physiologischer Liposomen zumindest teilweise auf ihrer Ähnlichkeit mit den Hüllstrukturen beispielsweise der Alphaherpesviren und Retroviridae begründet sein, so läßt sich prognostizieren, daß die Vertreter folgender Vertebraten-infizierender Virusfamilien mit physiologischen Liposomen therapierbar sind:
Herpesviridae (Alpha-, Beta-, Gammaherpesviren)
Togaviridae (z. B. Rötelnvirus)
Poxviridae (z. B. Pockenvirus)
Paramyxoviridae (z. B. Mumps-, Masernvirus)
Orthomyxoviridae (z. B. Influenza-Viren)
Coronaviridae (z. B. IBV)
Bunyaviridae (z. B. Hantaanvirus)
Arenaviridae (z. B. Lassa-Virus)
Retroviridae (z. B. HTLV-1, HTLV-2, HIV-1, HIV-2)
Flaviviridae (z. B. Gelbfiebervirus)
Rhabdoviridae (z. B. Tollwutvirus)
Hepadnaviridae (z. B. Hepatitis B-Virus)
Toroviridae (z. B. Berne Virus)
Filoviridae (z. B. Marburg Virus)

In einem Versuch gegen vermutlich durch Papillomaviren induzierte Warzen (Verrucae planae juveniles) konnte gezeigt werden, daß diese nach täglich mehrmaliger Applikation physiologischer Liposomen binnen drei Wochen verschwanden. Dieses Ergebnis sowie Erfolge bei Rhinovirusinfektionen (Picornaviridae) lassen jedoch auch sog. nackte Viren als mögliche Therapieziele erkennen:
Adenoviridae
Caliciviridae
Papovaviridae
Parvoviridae
Picornaviridae
Reoviridae
Iridoviridae
Birnaviridae

Möglicherweise können auch andere intrazelluläre Parasiten, die über einen ähnlichen Mechanismus wie Viren ihre Wirtszellen infizieren, durch physiologische Liposomen bekämpft werden. Zu derartigen Mikroorganismen zählen vor allem die Vertreter der Rickettsiaceae, Bartonellaceae, Chlamydiaceae und Mycoplasmataceae. Bei systemischer Applikation von Liposomen könnten auch einige der bisher als unheilbar geltenden virusinduzierten Autoimmun- und Tumorerkrankungen therapierbar werden.

Die unspezifische antivirale Wirkung physiologischer Liposomen läßt auf das unter Beispiel 1 aufgeführte Wirkmodell schließen und damit auf einen breiten Einsatz dieses Heilmittels gegen viele verschiedene Viren bei Pflanzen, Tieren und Mensch hoffen.

Auch als antiviraler Zusatz in bestimmten Bakterien- und Pilzkulturen, sowie in pflanzlichen und tierischen Zell- und Gewebekulturen sind physiologische Liposomen geeignet.

Langzeitversuche an Freiwilligen belegen, daß auch ein Einsatz physiologischer Liposomen als Prophylaktikum, z. B. gegen Herpes simplex Typ 1, von größter Wirksamkeit ist. Treffen einer oder mehrere der eingangs erwähnten antiviralen Wirkungsmechanismen physiologischer Liposomen zu, so dürften Resistenzen hiergegen - wie sie praktisch gegen alle gängigen antimikrobiellen Therapeutika im Laufe der Zeit auftreten - nur schwerlich von den Viren zu entwickeln sein.

### II. Verwendung der erfindungsgemäßen physiologischen Liposomen zur Prophylaxe und Therapie allergischer Reaktionen

Die erfindungsgemäßen Liposomen sind desweiteren zur lokalen und systemischen Inhibition allergischer Reaktionen von Mensch und Tier geeignet.

Wie allgemein bekannt, nehmen allergische Erkrankungen - zumindest in den Industriestaaten - von Jahr zu Jahr zu, wobei die Ursachen nicht völlig geklärt sind. Besonders häufig treten neben Schleimhautallergien, wie z. B. "Heuschnupfen", auch Hautallergien auf, hervorgerufen durch Kontakte mit z. B. Hefepilzen, Bakterien, Insekten, Hausstaub, Pollen, chemischen Substanzen und Arzneistoffen, sowie Hilfsstoffen in kosmetischen und pharmazeutischen Formulierungen u.v.a.m.. Eine häufige Ursache für lokal begrenzte Kontaktallergien ist das Tragen von Schmuck. Ein Großteil der Personen, die Schmuck tragen wollen, werden durch allergische Reaktionen daran gehindert. Besonders fallen in die Kategorie von "unverträglichem" Schmuck Stücke aus relativ leicht oxidierbaren Metallen (alle außer hochkarätigen Gold- und Platinlegierungen). Relativ selten treten aber auch Allergien gegen reines Gold auf. Die Unverträglichkeit, anders ausgedrückt, die Allergie beginnt in extremen Fällen bereits wenige Minuten nach Anlegen des Schmucks und äußert sich in Jucken der entsprechenden Hautstelle bis hin zu unerträglichen Schmerzen. Betroffenen Personen bleibt je nach Allergiegrad oft nur die Wahl, auf das Tragen von Schmuck gänzlich zu verzichten oder aber hochkarätigen Gold- oder Platinschmuck zu verwenden.

Eine ebenfalls weit verbreitete allergische Reaktion der Haut ist nach Bestrahlung mit ultraviolettem Licht bestimmter Wellenlänge zu beobachten. Die betroffenen Personen reagieren an den der Strahlung (Sonne, UV-Bräunung) ausgesetzten Hautpartien mit Erythem, Quaddelbildung und Schmerzen. Ein breites Vorkommen zeigen auch Nahrungsmittelallergien, wie z. B. gegen Gluten. Besonders häufig treten in den letzten Jahren allergische Reaktionen auf, die dem atopischen Formenkreis zugeordnet werden und die durch eine Vielzahl innerer und äußerer Faktoren hervorgerufen werden. Hierzu gehört vor allem die Neurodermitis. Zu den noch immer schwierig therapierbaren Krankheiten des allergischen Formenkreises zählt der durch Mediatorfreisetzung erzeugte Endotoxinschock.

Zur Behandlung solcher Allergien stehen Interna und Externa zur Verfügung. In der Regel werden Hautallergien mit Hilfe von Externa therapiert, in schweren Fällen werden zusätzlich Interna verordnet. Da oftmals die Ursache einer auftretenden Hautallergie unbekannt ist, wird eine sichere Diagnose ungemein erschwert. Das hat zur Folge, daß im Anfangsstadium mit sog. "harmlosen" Antiallergika therapiert wird, die oft nur zu einer Linderung der Beschwerden führen oder gar keine Wirkung zeigen. Der Arzt ist somit gezwungen, weiter zu therapieren und zu Dermatika, Antibiotika und Antimykotika (bei Sekundärinfektionen Allergie-geschädigter Hautpartien) zu greifen - also zu Arzneimitteln - , die Chloramphenicol, Rifampicin, Flumethason, Dexamethason, Triamincinolonacetonid oder Hydrocortison enthalten, um nur einige zu nennen. Viele dieser "harten" Arzneimittel haben Anwendungsbeschränkungen und können ihrerseits Nebenwirkungen hervorrufen, z. B. Hautatrophien, Steroidakne etc.. Von Dermatologen gefürchtet ist beispielsweise der Rebound-Effekt nach dem Absetzen von Cortison. Ferner führen sie oftmals zu einer Austrocknung der Haut und dürfen nicht mit der Bindehaut des Auges in Berührung kommen (siehe Abschnitt IV). Ein Problem, das bei der Verabreichung von externen Antiallergika auftritt, liegt in der unzureichenden Aufnahme durch die Haut. Die Penetration der Wirkstoffe ist besonders erschwert, wenn diese wasserlöslich sind. Ein vergleichbares Problem liegt vor, wenn der Wirkstoff lipophil ist und in Form einer Kristallsuspension aufgetragen werden muß. Die Effektivität externer Antiallergika gilt deshalb als unbefriedigend.

Ähnliche Probleme finden sich bei der Bekämpfung von Heuschnupfen. Um diese allergischen Reaktionen der Nasenschleimhaut nach Kontakt mit den entsprechenden Allergenen, wie z. B. Blütenpollen, Hausstaub, Substanzen von Tierhaaren usw., therapieren zu können, bedarf es ebenfalls der Anwendung "harter" Pharmaka, die häufig in Form eines Aerosols appliziert werden (z. B. Hydrocortison). Von einem prophylaktischen Einsatz derartiger Therapeutika ist jedoch aufgrund der zahlreichen Nebeneffekte abzuraten. Ein echtes, nebenwirkungsfreies Prophylaktikum lag bisher nicht vor.

Aus bereits vorhandenen Studien ist bekannt (1, 2, 3), daß Liposomen innerhalb kurzer Zeit (in einigen Fällen innerhalb weniger Minuten) tief in die Dermis der Haut einzudringen vermögen. In Schleimhäute dringen Liposomen binnen Sekunden ein. Somit kann von einer Tiefenwirkung lokal aufgetragener Liposomensuspensionen ausgegangen werden.

Der inhibitorische Effekt erfindungsgemäßer physiologischer Liposomen (ohne Wirkstoffzusatz) auf lokale allergische Reaktionen ist im Detail nicht geklärt, läßt sich aber vermutlich auf eines oder mehrere der folgenden Erklärungsmodelle zurückführen:
1. Liposomen, hergestellt aus Lipiden oder Lipoiden, z. B. aus Phosphatidylcholinen, dringen in Schleimhaut bzw. Dermis der Haut ein, füllen die interzellulären Räume auf und bringen die dort vorhandenen Zellen durch Fusion mit den Liposomen zum "Aufquellen". Dadurch wird das Gewebe möglicherweise "dichter" und verhindert so das Eindringen von Allergenen und die Verbreitung von Mediatoren. Vielleicht wird es dadurch auch widerstandsfähiger gegenüber mechanischer Belastung.
2. In die Haut bzw. Schleimhaut eingedrungene und daher auch in den interzellulären Räumen vorhandene Liposomen nehmen freigesetzte Mediatoren (z. B. vasoaktive Amine) auf und inhibieren dadurch deren Ausbreitung bis zu den Gefäßendothelzellen. Dadurch kommt es beispielsweise nicht zur Vasodilatation mit erhöhter Gefäßpermeabilität. Auch die in zahlreiche Membranprozesse involvierten Calciumionen werden von Liposomen aufgenommen und erst nach und nach wieder freigesetzt.
3. Liposomen interagieren durch Adsorption, Fusion und/oder Endozytose vermutlich auch mit den basophilen Granulozyten und Mastzellen und verändern möglicherweise deren Membranen (Plasmamembran oder auch intrazelluläre Membransysteme), so daß die Freisetzung von Mediatoren behindert wird.
4. Die in die unteren Haut- bzw. Schleimhautschichten vordringenden Liposomen verdrängen in den interzellulären Räumen befindliche Schadsubstanzen in das Lymph- und Gefäßsystem und/oder nehmen diese auf und beschleunigen so deren Metabolisierung (möglicherweise über Phagozytose mittels in der Haut vorkommender Zellen, wie z.B. Makrophagen).
5. Für den speziellen Fall der Allergien gegenüber bestimmten Metallen lassen sich die Metallionen-absorbierenden Eigenschaften von Liposomen als Erklärung für die beobachteten antiallergischen Wirkungen heranziehen: Bei Kontakt mit der Haut werden aus Metallen durch chemisch-physikalische Prozesse Atome bzw. Ionen in minimalen Mengen herausgelöst. Diese gehen meist eine Bindung mit körpereigenen Molekülen ein und wirken in diesen Verbindungen oftmals als Allergene (Hapten-Carrier-Komplex), welche die entsprechenden Abstoßungsreaktionen auslösen. Befinden sich Liposomen an denjenigen Hautstellen, die in Kontakt mit dem Allergen treten, so nehmen diese die freigesetzten Metallionen auf (eigene Untersuchungen), möglicherweise bevor diese an einen Carrier binden können. Die Liposomen selbst werden nach und nach von Körperzellen aufgenommen und metabolisiert. Die Metallionen werden durch zahlreiche intrazelluläre Detoxifizierungsmechanismen (z.B. Metallothionein) komplexiert und ausgeschieden.
6. Möglicherweise sind auch einige der Grundsubstanzen der Liposomen (essentielle Fettsäuren, wie z.B. Linolsäure) bzw. deren Stoffwechselprodukte als antiallergene Substanzen aktiv.
7. Versuche an mehreren Probanden haben gezeigt, daß vor allem Gallensäuren als Tenside zu sehr effektiven, antiallergischen Liposomen führen. Eine Erklärung könnte sich aus der möglichen Wirkung von Gallensäuren auf den intrazellulären cAMP-Spiegel und/oder die Ausschüttung von Prostaglandinen ergeben sowie auf physikalische Änderungen der Plasmamembran in Hinsicht auf ein verändertes Membranpotential. Die Wirkung von Histamin auf seine Zielzellen erfolgt über Bindung an spezielle Rezeptoren (H₁, H₂, H₃), die wiederum über assoziierte G-Proteine entsprechende Enzyme aktivieren (5). Die dabei gebildeten second messenger (cAMP, IP₃, DAG, Ca²⁺) aktivieren zahlreiche intrazelluläre Enzyme. Gallensäure oder Gallensäurederivate bzw. die durch sie ausgelösten Veränderungen der Zellen könnten einen oder mehrere der membranständigen Moleküle dieses Übertragungsweges beeinflussen bzw. deren Zusammenwirken stören.
8. Die antioxidativen Eigenschaften vor allem Vitamin C enthaltender Liposomen ermöglichen das Abfangen von Radikalen und nichtradikalen Noxen im inter- und extrazellulären Raum.

Die bis jetzt vorliegenden Ergebnisse lassen auf Hauptfaktoren für die Wirksamkeit physiologischer Liposomen schließen, wie sie unter den Punkten 1, 3, 7 und 8 beschrieben wurden.

Die vorliegenden Untersuchungen zeigen einen Allergie-inhibierenden Effekt der erfindungsgemäßen physiologischen Liposomen. Die hier beschriebenen Liposomen bestehen ausschließlich aus Substanzen biologischen Ursprungs, wie z. B. Phosphatidylcholin (Lecithin), woraus ein Großteil der Zellmembranen zumindest aller höheren Lebewesen besteht. Die hervorragenden antiallergischen Wirkungen dieser Liposomen erlauben erstmalig eine unbedenkliche, prophylaktische Anwendung in Form eines Kosmetikums bzw. Naturheilmittels und/oder prophylaktische bzw. therapeutische Anwendung als Pharmakon.

Die Erfindung stellt ein Mittel zur lokalen Applikation von Liposomen gegen Haut- und Schleimhautallergien sowie zur systemischen Applikation gegen Allergien des Verdauungstraktes und generalisierte Allergien (z. B. allergische Schockzustände) zur Verfügung. Die physiologischen Liposomen können je nach Bedarf mit beispielsweise Ascorbinsäure und anderen Antioxidantien bzw. Reduktionsmitteln kombiniert werden.

Erfindungsgemäß werden die Liposomen direkt als liposomale Suspension eingesetzt, vorzugsweise in der rein flüssigen Form, gegebenenfalls als Tropfen, Aerosol oder Infusionen, als Zusatz zu medizinischen Bädern oder auch mittels transdermaler therapeutischer Systeme, z. B. Pflaster, gegebenenfalls auch unter Zusatz von Gelbildnern oder anderen Andickungsmitteln oder ein- und mehrwertigen Alkoholen.

Physiologische Liposomen eignen sich hervorragend zur Bekämpfung von Haut- und Schleimhautallergien. Der Begriff der Schleimhautallergie bezieht sich zum überwiegenden Teil auf allergische Reaktionen der Nasen-, Mund- und Augenschleimhaut, die durch einen Kontakt mit entsprechenden Allergenen ausgelöst werden. Die dabei ablaufenden immunologischen Mechanismen gleichen denen der Hautallergien, die nachstehend am Beispiel der Ohrschmuckallergie erörtert werden. Der Begriff Hautallergie umfaßt Krankheitsbilder unterschiedlicher Genese, die jedoch alle zu einer ähnlichen, mehr oder weniger lokal begrenzten Hautreaktion (Allergie) führen.

Als ein Beispiel einer derartigen lokal auftretenden allergischen Reaktion soll die sehr häufig zu beobachtende Unverträglichkeit gegenüber Ohrschmuck dienen. Hier können zwei möglicherweise kausal unzusammenhängende Allergiereaktionen beobachtet werden: Zum einen eine spezifisch gegen bestimmte Substanzen (Allergene), wie Nickel, gerichtete Allergie, bei der die Patienten oftmals nur hochkarätigen Gold- oder Platinschmuck symptomlos vertragen; zum anderen eine Art mechanische Urtikaria (Druckurtikaria), bei der allein die mechanische Belastung des Ohrläppchens zu allergischen Reaktionen führt. Je stärker die mechanische Belastung durch Ohrstecker oder -clips ist, desto heftiger die Reaktion, wobei kleine Creolen bedingt durch geringes Gewicht und niedrigen Querschnitt des Ohr-durchdringenden Teils noch am besten vertragen werden. Selbstverständlich können beide Allergiereaktionen mit unterschiedlicher Gewichtung auch bei ein und demselben Patienten auftreten. Für den ersten Fall läßt sich als Mechanismus eine seit langem bekannte klassische Form der allergischen Reaktion angeben, bei der Antigen-spezifische IgE-Antikörper an der Oberfläche von in der Haut vorhandenen immunreaktiven Zellen, wie Mastzellen und basophilen Granulozyten, sitzen. Bei Antigenkontakt mit den zellständigen IgE-Antikörpern kommt es zu einer Signalgebung in das Zytoplasma dieser Zellen, was letztendlich eine Freisetzung (Degranulation) von Mediatoren aus den Speichervesikeln, wie z. B. Histamin, zur Folge hat. Die hierdurch hervorgerufene Vasodilatation in dem entsprechenden Areal führt zu Schwellung, Rötung und Erhitzung des Ohrläppchens. Da die Degranulation von Basophilen und Mastzellen bei 37°C - 38°C ihr Optimum erreicht, läuft dieser Vorgang durch die steigende Ohrtemperatur immer stärker ab (vgl. Abschnitt V). Durch die parallel verlaufende, erhöhte Gefäßpermeabilität vermehrt sich der Anteil der Gewebeflüssigkeit, was den Druck im Ohrläppchen weiter steigert. Dort vorhandene Nervenzellen registrieren diese Veränderungen mit entsprechender Schmerzempfindung. Der Übertritt von Blutplasmabestandteilen in das Gewebe kann dabei so stark sein, daß bei einigen Patienten (auch ohne Ohrloch!) massiv Blutplasma durch die Hautporen des Ohrläppchens austritt. Die Symptome klingen nur sehr langsam ab, das Ohr bleibt oftmals über Wochen druckempfindlich. In extremen Fällen kommt es zu nekrotischen Prozessen, die permanente Veränderungen am Ohrläppchen zur Folge haben. Bei der mechanischen Urtikaria ist kein spezielles Antigen beteiligt, um letztendlich die gleichen Symptome, wie eben geschildert, hervorzurufen. Es kann angenommen werden, daß ein massiver Besatz hautständiger Basophiler und Mastzellen und/oder in ihrer Degranulation relativ instabiler Basophiler und Mastzellen allein durch mechanische Einwirkung (Druck) ihre Mediatoren freisetzen und so die o. g. Reaktionen hervorrufen. Da unter normalen Bedingungen hormonelle physiologische Antagonisten, wie Catecholamine, die Freisetzung von Mediatoren unterdrücken, lassen sich auch die zeitlichen Schwankungen in der Empfindlichkeit einzelner Patienten erklären. Unter Streßbedingungen, wenn die natürliche hormonelle Kontrolle der physiologischen Antagonisten gestört ist, kommt es daher häufig zu stärkeren allergischen Reaktionen.

Als Anwendungsgebiete der vorliegenden Erfindung gelten alle Bereiche, bei denen durch einen bevorstehenden körperlichen Kontakt eines Patienten eine Allergie, wie Urtikaria, ekzematöse Dermatitis oder Schleimhautallergie, zu erwarten ist (Prophylaxe); z.B.:
- in der Medizin bei Verwendung von: Pflaster, Verbandsmittel, topische Arzneimittel, Elektroden, Sensoren, kurzwellige elektromagnetische Strahlung, Wundverschlüsse (Klammern), Infusionskatheder, Prothesen und eventuell auch Hauttransplantationen mit z. B. Hautersatzstoffen
- in der Zahnmedizin bei Verwendung von: Provisorien, Zahnersatz
- im "Alltag" bei Kontakt mit: bestimmten Nahrungsmitteln, Pflanzenpollen, Substanzen von Tierhaaren oder -häuten, Hausstaub, Schmuck, Brillen, Uhren, Hörgeräten, Kleidung, Waschmitteln, Kosmetika, UV-Licht u. a..
- als gewerblicher Hautschutz

Als weitere Therapiebereiche zählen mit allergischer Ätiologie einhergehende Erkrankungen, wie beispielsweise das atopische Ekzem/Neurodermitis. Ferner gelten als weitere Anwendungsgebiete systemische Therapien generalisierter allergischer Reaktionen, wie beispielsweise allergische Schockzustände.

Erste Untersuchungen an freiwilligen Testpersonen zeigten, daß eine einmalige Applikation physiologischer Liposomen in bisher allen Fällen eine mehrstündige bis tagelange Unterdrückung der allergischen Reaktion gegen Ohrschmuck erzeugt. Die Testung physiologischer Liposomen in Form von Aerosolen zeigte bei einem Teil der Probanden mit Heuschnupfen eine extreme Verbesserung des allergischen Zustandes, oftmals führte die liposomale Applikation zu einer vollständigen Inhibierung der Allergie (siehe Beispiel 2). Ähnlich positive Ergebnisse wurden bei Untersuchungen gegen Sonnenallergie, Kontaktallergien (u. a. gegen Arm-, Finger- und Halsschmuck, Uhren), allergisch bedingte Ekzeme, wie z. B. Erythema exsudativum multiformis, und Neurodermitis, erzielt. Ein Patient mit hochgradigem endogenen Ekzem, bei dem die Corticoidtherapie aufgrund des sofort immer wieder auftretenden Rebound-Effektes erfolglos blieb, ist nach Behandlung mit physiologischen Liposomen seit einem Jahr symptomfrei. Ascorbinsäure enthaltende Liposomen erwiesen sich bei diesen Untersuchungen oftmals als besonders effektiv, was auf eine Beteiligung der als Mediatoren freiwerdenden Sauerstoffradikale hinweist.

### III. Verwendung erfindungsgemäßer physiologischer Liposomen zur Prophylaxe und Therapie von Schmerzen und Muskelverspannungen

Die erfindungsgemäßen Liposomen können ferner zur Behandlung tonischer Schmerzen und den damit zusammenhängenden Folgesymptomen eingesetzt werden. In den getesteten effektiven Konzentrationen konnten keinerlei negative Wirkungen der Liposomen festgestellt werden. Für die Therapie mit erfindungsgemäßen physiologischen Liposomen sind hauptsächlich die an erster Stelle der Schmerzgenerierung stehenden Nozizeptoren, die morphologisch am wenigsten differenzierten Rezeptoren, von Bedeutung. Diese nicht-myelinisierten Nervenendigungen stellen die Sinnesorgane für Schmerz in fast allen Geweben dar. Sie reichen beispielsweise in der Epidermis bis an das Stratum lucidum, also dicht unter die Hornhautschicht, heran. Zwei in ihrer Leitungsgeschwindigkeit unterschiedliche Fasersysteme (Aδ- und C-Fasern) leiten die Schmerzimpulse zum Zentralen Nervensystem. Das Vorhandensein eines schnell- und eines langsamleitenden Systems erklärt die zwei beobachteten Schmerzarten: Der erste Schmerzreiz verursacht eine "helle", durchdringende, gut lokalisierbare Empfindung, auf die ein "dumpfer", bohrender, schwer lokalisierbarer Schmerz folgt. Auslösende Ursachen für den sog. Nozizeptorschmerz sind Gewebeläsionen, die durch pathologische Veränderungen im Inneren entstehen. Dabei kommt es zu einer Veränderung (z. B. erhöhte Zellmembraninstabilität) oder Zerstörung von Zellen, wodurch algogene Substanzen freigesetzt werden. Sehr wichtig bei diesem akuten Geschehen ist die Bildung von Prostaglandin E₂, welches die Nozizeptoren für die algogenen Substanzen sensibilisiert. Algogene Stoffe, wie Histamin, Kalium, Kinine, Serotonin und Substanz P, werden entweder über Ionenkanäle ausgeschüttet (z. B. Kalium) oder exozytiert (z. B. Histamin). Hierin gleichen sich die Anfangsschritte bei der Schmerzgenerierung und der Allergieerzeugung. Bei manchen Substanzen wie Histamin, Prostaglandinen und Kininen läßt sich von einem geradezu identischen Initiationsvorgang sprechen (siehe Abschnitt II). Dies dürfte auch die hauptsächliche Erklärung für eine Wirkung physiologischer Liposomen auf beide Krankheitsformen sein.

Bis heute existieren keine Analgetika, die nicht unerwünschte Nebenwirkungen hervorrufen. Als Beispiel seien hier nur die mögliche Atemlähmung und die Suchtgefahr bei opioidhaltigen Analgetika sowie die Schädigungen der Magendarmschleimhaut bei Salicylsäurederivaten aufgeführt. Bedingt durch die Komplexität der in Schmerzerzeugung und -verarbeitung involvierten Prozesse existieren viele, völlig unterschiedliche Ansatzpunkte, um eine Analgesie zu erzeugen (hier sind nur die gängigsten Mechanismen angeführt; (5)):
- Corticosteroide hemmen die Bildung von Arachidonsäure und damit dessen Metabolisierung zu Prostaglandin E₂
- nicht-steroidale Antiphlogistika hemmen die Bildung cyclischer Endoperoxide und insofern auch die Entstehung von Prostaglandinen
- Opioidanalgetika unterdrücken die Schmerzempfindung durch einen Angriff an die zentralen Schaltstellen des nozizeptiven Systems
- antiphlogistische Analgetika wirken einerseits direkt im Bereich der Nozizeptoren im geschädigten Gewebe, andererseits aber auch zentral, vermutlich ebenfalls durch eine Hemmung der Prostaglandinsynthese
- antipyretische Analgetika wirken möglicherweise ähnlich wie antiphlogistische Analgetika über eine Hemmung der Prostaglandinsynthese.

Alle hierfür verwendeten Substanzen generieren mehr oder weniger starke unerwünschte Nebenwirkungen (wie zuvor erwähnt), da sie in physiologisch bedeutsame Vorgange eingreifen.

Durch Testung an Probanden mit unterschiedlichen Symptomen läßt sich die analgetische Wirkung physiologischer Liposomen nachweisen, wobei sich Vitamin C enthaltende Liposomen manchmal als effektiver erweisen (evtl. Modulation des Prostaglandinmetabolismus durch Vitamin C oder andere Wirkungen (siehe 11)).

Wie schon in den Abschnitten I und II beschrieben, verschwinden die durch Virusinfektion oder Allergie ausgelösten Schmerzen nach Applikation der Liposomen in den meisten Fällen sofort. Zumindest bei einem Teil der Mediatoren, die durch virusbedingte Gewebeläsionen oder allergische Reaktionen ausgeschüttet werden, handelt es sich auch um algogene Substanzen, welche die Nozizeptoren des betreffenden Gewebes alarmieren. Eine objektive Beurteilung, die eine Einwirkung physiologischer Liposomen auf Nervenaktivität nachweist, ergibt sich aus den in Beispiel 3 ermittelten Befunden am isolierten Darm des Meerschweinchens. Die deutliche Inhibierung der Nervenaktivität bei relativ niedrigen Liposomenkonzentrationen beweist die überraschende Wirksamkeit der Cholat-Liposomen. Die als Kontrolle verwendeten Cholat-freien Liposomen führen zu einer Stimulation der Nerven. Da durch die relativ geringe Menge an Liposomen im Organbad ein Einfluß der Liposomen auf die Ionenzusammensetzung der Flüssigkeit vernachlässigbar gering ist, Cholat-freie Liposomen jedoch eine derart deutliche Stimulation bewirken, muß davon ausgegangen werden, daß auch diese Liposomen mit dem Gewebe bzw. den Zellen direkt interagieren. Aus zahlreichen Experimenten ist bekannt, daß Tensid-haltige Liposomen einen größeren Verlust eingeschlossener hydrophiler, kleiner Ionen oder Moleküle aufweisen als Tensid-freie Liposomen. Auch die hier verwendeten Cholat-Liposomen zeigen diese Eigenschaft; d. h. Tensid-haltige Phospholipidmembranen besitzen eine geringfügig erhöhte Durchlässigkeit (in beiden Richtungen) für derartige Substanzen. Wenn Liposomen mit relativ hohem Cholatanteil (wie die hier verwendeten) mit Zellen fusionieren, so überträgt sich durch die miteingebrachten Tenside die leicht erhöhte Membrandurchlässigkeit, beispielsweise für Ionen wie Na⁺ und K⁺, auf die Zellmembranen. Durch den nunmehr verstärkten Ionenfluß verschiebt sich das Membranpotential (Ruhepotenial) in Richtung auf die "Zünd-Schwelle" (firing level) (10). Dadurch steigt zwar die Erregbarkeit der Zelle, gleichzeitig bewirkt jedoch die der Depolarisation entsprechende Verkleinerung des Aktionspotentials eine Verminderung der freizusetzenden Überträgersubstanzen (es werden weniger Vesikel ausgeschüttet) und dadurch eine verringerte Erregung der postsynaptischen Zelle (4). Diese präsynaptische Wirksamkeit konnte in weiteren Experimenten mit Acetylcholin- bzw. Histamin-stimuliertem isolierten Meerschweinchendarm bestätigt werden. Für eine Fusion Tensid-freier Liposomen mit Zellen ergibt sich hingegen eine Änderung des Membranpotentials weg von der "Zünd-Schwelle", was demzufolge ein vergrößertes Aktionspotential und eine verstärkte Freisetzung (erhöhte Vesikelausschüttung) von Überträgersubstanz nach sich zieht. Dies führt zur beobachteten Stimulation der Darmkontraktion.

Das hier vorgestellte Modell geht im Gegensatz zu den zuvor dargestellten Therapieansätzen zur Schmerzreduktion davon aus, daß sowohl die Mediatorausschüttung inhibiert als auch die Membranen der Nozizeptoren durch Fusion mit physiologischen Liposomen "stabilisiert" werden bzw. ihr Ruhepotential in Richtung "Zünd-Schwelle" verschoben wird. Auch eine Wechselwirkung mit Schmerz-dämpfenden Rezeptoren, wie etwa den Opioidrezeptoren, kann die analgetischen Wirkungen physiologischer Liposomen erklären. Ob dabei die Rezeptoren selbst, die diesen Rezeptoren zugehörigen, membranständigen G-Proteine oder die ebenfalls an der Signalübersetzung beteiligte Adenylatcyclase (5) durch Liposomenbestandteile oder die den Fusionen folgenden physikalischen Änderungen beeinflußt oder aber die räumliche Anordnung dieser Strukturen in der Zellmembran zueinander verändert werden, bedarf weiterer Untersuchungen. Die Beeinflussung eines oder mehrerer dieser Faktoren würde dabei zu einem erniedrigten cAMP-Spiegel führen, der wiederum zahlreiche zellphysiologische Konsequenzen nach sich zieht (5). Möglicherweise kommen aber beide Wirkungsmechanismen gleichzeitig zum Tragen. Die analgetische Wirkung physiologischer Liposomen läßt sich aber auch bei Schmerzen beobachten, bei denen keine sichtbare Beeinträchtigung des Gewebes vorliegt. So können Schmerzen, die kontinuierlich oder intervallartig an oder in der Nähe von früheren Verletzungen (Brüche, Operationsnarben usw.) oder als sog. Weichteilrheumatismus (Fibromyalgie) auftreten, durch ein- oder mehrmaliges Auftragen (je nach Patient) von Liposomen in kürzester Zeit zum Verschwinden gebracht werden. Dies würde bedeuten, daß derartige Schmerzen durch die in der Epidermis vorhandenen Nozizeptoren erzeugt werden. Eine Erklärung findet dieses Phänomen anhand der sog. Konvergenz-Projektions-Theorie, wonach Erkrankungen der Tiefenstrukturen und Eingeweide manchmal Schmerzen auf Bereiche der Körperoberfläche projektieren, die demselben oder benachbarten Segmenten angehören wie das erkrankte Organ. Ebenso werden Schmerzen bei Alkoholpolyneuropathie und diabetischer Neuropathie bekämpft, möglicherweise effektiver unter Zusatz geringer Mengen an Calciumionen und Vitaminen des B-Komplexes.

Besonders gravierend zeigt sich die Wirkung physiologischer Liposomen auf Muskelverspannungen und Schonhaltungen (vgl. Beispiel 3), wie sie beispielsweise bei der sog. Nackensteifigkeit und einigen ischiadiformen Beschwerden auftreten. Diese Symptome beruhen auf einer tonischen Reflexaktivität, welche durch eine chronische Schädigung verursacht wird. Durch den häufigen Zug an Bändern, Sehnen und Gelenken entstehen Gewebeschädigungen, die aufgrund der Ausschüttung algogener Substanzen die nozizeptive Reflexaktivität erhöhen. Der Körper versucht daraufhin durch verstärkte Muskelaktivität diese vorgeschädigten Bereiche zu entlasten (verkrampfte Schonhaltung) und gerät dabei in einen Circulus vitiosus. Bei der Applikation physiologischer Liposomen am Beispiel der Nackensteifigkeit zeigt sich, daß der stechende Schmerz, der bei Verdrehen des Kopfes zur Seite der Muskelverspannung hin entsteht, nach einmaligem Auftragen von 2 - 5 ml Vitamin C enthaltender Liposomen auf Nacken und Schulterbereich nach etwa einer Minute abklingt. Der Kopf läßt sich wieder schmerzfrei zur Seite bewegen, wenn auch die Muskelverspannung (meist im Bereich des Kapuzenmuskels) vorläufig bestehen bleibt. Diese Verspannung löst sich aber nach mehrmaliger Applikation meist binnen eines Tages. Bisher konnten derartige Beschwerden nur durch mehrmalige intramuskuläre Injektion eines Analgetikums (z. B. Lidocain) und oraler Verabreichung von Muskelrelaxantien nach mehreren Tagen behoben werden. Vitamin B₁₂ enthaltende Liposomen führten in einem getesteten Fall mit sogenanntem Tennisarm - mit Beschwerden also, bei denen es bereits zu entzündlichen Schädigungen gekommen ist - binnen einer Woche bei täglicher Applikation zur Beschwerdefreiheit.

Als ein weiteres Beispiel sei hier die Behandlung der sog. Zahnhalsempfindlichkeit angeführt, welche die Patienten sehr sensibel vor allem gegenüber Kälte, Hitze, Süße und Säure macht. Ursache hierfür ist häufig ein mehr oder weniger stark freiliegender Zahnhals, welcher, z. B. durch Säureschäden im Bereich der Incisivi zusätzlich verbreitert, die Nozizeptoren innerhalb des Zahnes nur unvollständig von der "Außenwelt" isoliert. Durch das poröse Dentin gelangen chemische Noxen bzw. Temperaturdifferenzen nur wenig abgeschwächt an die Nozizeptoren und erzeugen Schmerzen. Die erste Applikation physiologischer Liposomen (Spülung oder Aufsprühen) erzeugt häufig einen kurzen stechenden Schmerz, welcher möglicherweise auf eine Membranpotentialveränderung der Nozizeptoren durch Fusion mit Liposomen hinweist. Der Schmerz wird bei weiteren Applikationen stufenweise geringer und verschwindet schließlich ganz. Dieses Abklingen geht einher mit einer zunehmenden Unempfindlichkeit gegenüber Kälte, Hitze, Süße, Säure usw.. Schließlich genügt eine ein- bis zweimalige Anwendung pro Tag zur Behandlung der Zahnhalsempfindlichkeit. Sehr rasch analgetisch wirken physiologische Liposomen auch bei Hautverletzungen, wie etwa Läsionen des Nagelbettes. Weitere Beispiele für die analgetischen Eigenschaften physiologischer Liposomen finden sich unter Abschnitt V.

Die gegenüber herkömmlichen Analgetika nebenwirkungsfreie Therapie favorisiert physiologische Liposomen für eine breite Verwendung vor allem gegen heute nur mit "harten" Pharmaka therapierbare Symptome wie beispielsweise Weichteilrheumatismus und Muskelverspannungen.

### IV. Verwendung erfindungsgemäßer physiologischer Liposomen zur Prophylaxe und Therapie von Augenerkrankungen

Die erfindungsgemäßen Liposomen können weiter prophylaktisch und therapeutisch bei verschiedenen Erkrankungen des Auges, wie beispielsweise allergisch oder viral bedingten Entzündungen, Irritationen aufgrund mangelnder oder physiologisch ungünstig zusammengesetzter Tränenflüssigkeit und Schäden durch chemische und physikalische Einwirkungen, eingesetzt werden.

Viele entzündliche Erkrankungen am Auge werden durch Allergene oder Viren ausgelöst und führen - durch bakterielle Superinfektionen verstärkt - zu oftmals permanenten Beeinträchtigungen. Wie empfindlich die Gewebe am Auge sind, zeigt sich schon daran, daß viele Allgemeinleiden (z. B. Schnupfen) Augenkrankheiten (z. B. Konjunktivitis) nach sich ziehen. Die Probleme bei der derzeitigen Therapie allergischer und viraler Erkrankungen wurden bereits in den Abschnitten I und II beschrieben, jedoch treten sie bei der Anwendung am Auge verstärkt hervor. So dürfen die bei entzündlichen Prozessen häufig applizierten Corticosteroide nicht eingesetzt werden, wenn eine Schädigung des Hornhautepithels vorliegt, da es sonst zu einer schnellen, progredienten Geschwürsbildung und evtl. Perforation mit Verlust des Auges kommen kann. Einige Antiallergika führen zu einer Verringerung des Tränenflusses und generieren dadurch neue Probleme. Die gegenwärtig am Auge zugelassenen Virostatika (z. B. Aciclovir) können, bedingt durch ihre starken Nebenwirkungen, nur im Notfall appliziert werden. Die häufig auftretenden Irritationen aufgrund mangelnder oder physiologisch ungünstig zusammengesetzter Tränenflüssigkeit (Dry-Eye-Symptomatik) können derzeit nur mit Tränenersatzmitteln behandelt werden. Deren umständliche Applikation (Augentropfen mehrmals täglich) sowie ihre immer mangelhafte Wirkung haben bei der Therapie dieser Symptome noch keinen echten Durchbruch erzielen lassen. Schäden durch chemische und physikalische Einwirkung, wie beispielsweise Verätzungen und Verbrennungen, stellen nach wie vor schwerwiegende therapeutische Probleme dar. Eine wirkliche, die Regeneration der Bindehäute und Epithelien fördernde Therapie steht derzeit nicht zur Verfügung. Viele Menschen müssen auf das Tragen von Kontaktlinsen verzichten, da ihre Augen diesen Fremdkörper nicht dulden. Wenn sie an einem der nachfolgenden Symptome leiden, kommen Kontaktlinsen als Sehhilfen ohnedies nicht in Frage: Keratoconjunctivitis sicca, Sjögren-Syndrom, Störungen der Muzinschicht (Stevens-Johnson-Syndrom, Verätzungen, Vitamin A-Avitaminose und okuläres Pemphigoid) und reduzierte Hornhautsensibilität. Ferner führen verringerte Tränenflüssigkeit (z. B. nach Einnahme von Antidepressiva, Antihistaminika, Diuretika und Spasmolytika), chronische Entzündungen des Auges (Blepharitis, Konjunktivitis usw.) sowie Aufenthalt bei niedriger Luftfeuchtigkeit (z. B. Flugpersonal) oftmals zu Problemen mit Kontaktlinsen. Die meisten dieser Ursachen lassen sich heute therapeutisch nicht so weit beheben, daß Kontaktlinsen problemlos auf Dauer vertragen werden. Der ohne chirurgischen Eingriff zur Blindheit führende Graue Star könnte - sollten sich die Ergebnisse von Experten der Tufts Universität in Boston bestätigen (6) - durch hohe Dosen Vitamin C (800 mg pro Tag) zumindest teilweise vermieden werden. Danach sollte die antioxidative Wirkung von Vitamin C für die Hemmung der altersbedingten Oxidation der Linse verantwortlich sein. Viele Menschen vertragen aber derart hohe Mengen Vitamin C oral nicht. Daher wäre es wünschenswert, physiologisch verträgliche, antioxidative Substanzen in die unmittelbare Nähe der Augenlinse heranzubringen. Derzeit steht jedoch kein derartiges Therapeutikum zur Verfügung.

Wie bereits in den Abschnitten I und II beschrieben, verfügen die erfindungsgemäßen physiologischen Liposomen über antivirale und antiallergische Eigenschaften, die ihren Einsatz als Ophthalmikum nahelegen. Außer diesen therapeutischen Ansätzen kommen in diesem speziellen Fall vor allem regenerationsunterstützende (siehe Abschnitt I, Punkt 8) und protektive Fähigkeiten physiologischer Liposomen hinzu. Der vordere Teil des Auges, vor allem die Cornea sowie die daran anliegenden Bindehäute, werden von einem permanenten Flüssigkeitsfilm überzogen. Diese Tränenflüssigkeit beinhaltet neben Nährstoffen, Salzen und antimikrobiellen Substanzen auch Stoffe, die eine rasche Verdunstung verhindern (z. B. Lipide und Muzine). Die Komposition der die Cornea und Bindehäute benetzenden Tränenflüssigkeit ergibt sich aus der Sekretion verschiedener Drüsen, wobei die Tränendrüse den Hauptteil der Flüssigkeit sekretiert. Im Augenlid selbst sitzen alveoläre Talgdrüsen (Meibom'sche Drüsen), apokrine Drüsen (Mol'sche Drüsen) sowie kleine akzessorische Tränendrüsen (Krause'sche Drüsen). Die bradytrophe Cornea, speziell das vordere Cornea-epithel (5-6 Schichten unverhornte Epithelien) wird durch die Tränenflüssigkeit über Diffusion ernährt. Dies erklärt die besondere Sensibilität der Cornea gegenüber Störungen der Tränenflüssigkeit. Wie im Beispiel 4 anhand einer Patientengruppe mit Dry-Eye-Symptomatik nachgewiesen wird, können physiologische Liposomen - außen auf das Augenlid aufgebracht - in vielen Fällen zu einer Normalisierung der die Cornea und Bindehäute benetzenden Flüssigkeitsschicht beitragen. Die Liposomen dringen vermutlich dabei durch das sehr dünne, mehrschichtige, verhornte Plattenepithel der Augenlidvorderseite ein und liefern so den lidständigen Drüsen Flüssigkeit, Nährstoffe und sekretierbare Substanzen (Lipide), welche die Verdunstung der Tränenflüssigkeit verlangsamen. Ferner zeigen Studien an Probanden mit allergen- und virusbedingten Konjunktivitiden sowie Keratiden, daß nach Auftragen physiologischer Liposomen auf das Augenlid das "Sandkorngefühl" binnen weniger Minuten verschwindet und in den meisten Fällen die Symptome bei mehrmaliger Applikation innerhalb eines Tages abklingen.

Physiologische Liposomen eignen sich demzufolge zur Prophylaxe und Therapie von Augenerkrankungen allergischer und viraler Ätiologie wie beispielsweise:
- Bindehautphlyktäne
- Blepharitis
- Chemose
- Dakryoadenitis
- Episkleritis, Skleritis und Tenonitis
- Herpes corneae, Herpes simplex der Lider und Herpes zoster ophthalmicus
- Iritis und Iridozyklitis
- Keratitis
- Konjunktivitis, wie C. follicularis, C. allergica, C. epidermica, C. vernalis, Einschlußblennorrhö und Schwimmbadkonjunktivitis
- Molluscum contagiosum
- Ophthalmia sympathica

Da Gallensäuren antibakteriell wirken, zeigen die erfindungsgemäßen Cholat-Liposomen auch bakterizide Wirkungen, zumindest gegen cholat-sensible Bakterien; d.h., es sind auch einige Infektionen bakterieller Ätiologie bei o. g. Krankheiten therapierbar.

Wegen der regenerationsunterstützenden und protektiven Wirkungen können physiologische Liposomen auch gegen folgende Symptome eingesetzt werden:
- Dry-Eye-Symptomatik
- Fädchenkeratitis
- Irritationen aufgrund von Verätzungen, Verbrennungen, Bestrahlungen und nach operativen Eingriffen
- Keratoconjunktivitis sicca
- Sjögren-Syndrom
- Stevens-Johnson-Syndrom
- Kontaktlinsenunverträglichkeiten aufgrund o. g. Ursachen

Ein Zusatz von Nährstoffen oder Mucoproteinen zu den Liposomen kann sich bei besonders schwerer Symptomatik als wirkungsvoller erweisen. Mit antioxidativen Substanzen, wie beispielsweise Vitamin C, beladene physiologische Liposomen können möglicherweise als Prophylaktikum gegen bestimmte Formen des Grauen Stars (z. B. Cataracta senilis) appliziert werden.

### V. Verwendung der erfindungsgemäßen physiologischen Liposomen zur Prophylaxe und Therapie von Entzündungen und Gewebeläsionen

Die erfindungsgemäßen Liposomen sind auch lokal und systemisch als Antiphlogistikum und als Mittel gegen Zell- und Gewebeläsionen anwendbar.

Wie schon in den Abschnitten II und III dargestellt, dürfte einer der Hauptwirkungsmechanismen physiologischer Liposomen die Inhibierung der Mediatorfreisetzung und die Neutralisierung freigesetzter Mediatoren sein. Die verstärkte Wirkung durch Vitamin C enthaltende Liposomen deutet auf pathologische Effekte oxidierender Substanzen bei diesen Prozessen hin. In jüngster Zeit wird sauerstoffradikalen hierfür eine immer größere Bedeutung beigemessen (5). Die wichtigsten Vertreter dieser Radikale sind das Superoxid-Radikal-Anion, das Hydroxyl-Radikal, Alkoxy-Radikale und Peroxy-Radikale. Pathogenetisch relevant sind aber auch nichtradikalische Spezies wie Singulettsauerstoff, organische Peroxide und Wasserstoffperoxid, sowie unterchlorige Säure, Chlor und Chloramine, die biologisch aus reaktiven Sauerstoffspezies entstehen. Derartig hochreaktive Substanzen fallen sowohl während normaler physiologischer Vorgänge als auch verstärkt als antimikrobielle Abwehr bestimmter Immunzellen an. Um sich selbst zu schützen, stehen den Zellen verschiedene Enzyme zur Verfügung, von denen die Superoxiddismutase und Katalase die wohl wichtigsten sind. Daneben wirken auch die Vitamine E, C und A als Antioxidantien. Die beiden äußerst effektiven Enzyme liegen jedoch fast ausschließlich intrazellulär vor, wohingegen der extra- bzw. interzelluläre Raum, in den hinein polymorphkernige Leukozyten, Monozyten und Makrophagen die reaktiven Sauerstoffspezies entlassen, fast gänzlich frei von entsprechenden Detoxifizierungsmechanismen ist. Dort schädigen diese hochreaktiven Substanzen nicht nur die eingedrungenen Mikroorganismen, sondern auch das eigene Gewebe. So kommt es bei den subzellulären Strukturen zu
- Depolymerisation von Collagen, Proteoglykanen und Hyaluronsäure
- Zersetzung von Lipiden
- Denaturierung von Enzymen
- Inaktivierung von Serinprotease-Inhibitoren (α₁-Antitrypsin)
- Bildung leukotaktischer Faktoren durch Interaktion mit Metaboliten der Arachidonsäure.

Als Einwirkungen auf zelluläre Strukturen könnten folgende Effekte bei entzündlichen Prozessen eine wichtige Rolle spielen:
- (Selbst-)Zerstörung von Leukozyten
- Erhöhung der Gefäßpermeabilität
- Erythrozytenlyse durch Schädigung von Membranlipiden.

Besondere Bedeutung kommt den leukotaktischen Faktoren zu, die weitere Phagozyten zum Ort der Entzündung rekrutieren und somit eine weitere Steigerung der Menge an reaktiven Sauerstoffspezies erzielen. Als entscheidend für die erste Rekrutierung von Phagozyten in geschädigtem Gewebe wird Interleukin 8 betrachtet. Als weitere chemotaktische Mediatoren dienen dann Leukotrien B₄ und die aktivierte Komplementkomponente C5a, das bedeutendste der drei Anaphylatoxine (C3a, C4a, C5a). Die außerordentlich hohe Wirksamkeit dieser chemotaktischen Substanz läßt sich daran erkennen, daß bereits eine Konzentration von 1 - 5 nM zu einer Rekrutierung weiterer polymorphkerniger Leukozyten führt (5). Mit dieser Chemotaxis erfolgt gleichzeitig eine Aktivierung der Leukozyten, die zu einer Freisetzung lysosomaler Enzyme und Bildung weiterer reaktiver Sauerstoffspezies führt. Da C5a auch auf die Gefäßendothelien einwirkt, kommt es zu Leukozytenaggregation, Leukozytenadhärenz an den Endothelien und schließlich zu einem Durchwandern der Gefäßwand. Auch dabei sind sekundäre Mediatoren beteiligt. An Blutplättchen wirkt C5a proaggregatorisch. Die Ausbreitung dieses Prozesses kann auf lokale Entzündungsprozesse beschränkt bleiben oder aber zu einer systemischen Anaphylatoxinbildung führen, die in einem lebensbedrohlichen Schock endet. Zu den Vorgängen, die zu einer systemischen Komplementaktivierung mit Anaphylatoxinbildung führen können, zählen: Endotoxinämie, Bakteriämie, Polytraumata, Verbrennung, Immunkomplex-Allergie, Hämodialyse, Leukopherese, Kardiopulmonärer Bypass und radiographische Kontrastmittel. Ein durch die systemische Anaphylatoxinbildung ausgelöstes Krankheitsbild ist die Schocklunge ("adult respiratory distress syndrome"), bei der pathogenetisch nicht nur die Aggregation der Leukozyten, sondern auch deren mit dem Auftreten reaktiver Sauerstoffspezies verbundene Aktivierung und das Auftreten sekundärer Mediatoren aus der Arachidonsäurekaskade eine Rolle spielen. Die Beteiligung von Anaphylatoxinen an lokalen Entzündungen setzt eine Komplementaktivierung im Gewebe oder in der interstitiellen Flüssigkeit voraus. Das Vorhandensein von Komplementkomponenten in der Lymphe sowie die Extravasation von Plasma ins Gewebe mittels anderer Entzündungsmediatoren lassen eine lokale Komplementaktivierung und damit eine Anaphylatoxinbeteiligung an lokalen Entzündungsprozessen möglich erscheinen. C5a wurde in Synovialflüssigkeit rheumatischer Patienten, entzündlichen Exsudaten, in Gefäßen mit immunologisch bedingten Entzündungen und auch im Liquor cerebrospinalis bei Meningitis nachgewiesen (5). So kann die leukozytäre Infiltration bei derartigen Entzündungen auf der chemotaktischen Wirkung von C5a beruhen, die dann über Aktivierung polymorphkerniger Leukozyten durch Freiwerden lysosomaler Enzyme (z. B. leukozytärer Elastase) oder Bildung reaktiver Sauerstoffspezies Gewebeschäden hervorruft. Sehr vereinfacht ausgedrückt, läßt sich folgendes Bild entwerfen: Fremdsubstanzen oder Noxen verleiten bestimmte Immunzellen zur Freisetzung reaktiver Sauerstoffspezies, es kommt zur Bildung von C5a, es folgt eine Rekrutierung weiterer Immunzellen mit Freisetzung von noch größeren Mengen an reaktiven Sauerstoffspezies usw.. Die Reaktion kann auch gleich mit der Bildung von C5a beginnen, wenn die Noxe entsprechend stark einwirkt, wie beispielsweise eine Verbrennung. Bei einem Eingriff an irgendeiner Stelle dieser Kaskade ließe sich das Aufschaukeln dieser Reaktion vermeiden.

Die therapeutischen Möglichkeiten, die sich aus dem Wissen um die reaktiven Sauerstoffspezies ergeben, sind noch weitgehend ungenutzt. Einer der wenigen Ansätze ist die Applikation von boviner Superoxiddismutase (Peroxinorm®). Obwohl aufgrund ausgeprägter Homologie zum menschlichen Enzym das immunogene Potential dieser Rinder-SOD beim Menschen relativ gering ist, bleibt die Therapie auf lokale Injektionen oder Infiltrationen beschränkt. Hierbei wurden nur selten lokale oder generalisierte allergische Symptome beobachtet, jedoch bleibt eine intravasale Applikation kontraindiziert. Sofern durch lokale Applikation hinreichend hohe Gewebespiegel erzielt werden können, werden mit SOD therapeutische Erfolge erzielt (z. B. bei Gonarthrosen, rheumatoider Arthritis, Strahlenzystitis, interstitieller Zystitis und induratio penis plastica). Seit kurzem wird auch eine humane Superoxiddismutase angeboten, deren Einsatz das Behandlungsspektrum sicherlich erweitern wird. Interessant in diesem Zusammenhang sind Erkenntnisse aus der Gerontologie, wonach es zumindest im Tiermodell Korrelationen zwischen besonders langlebigen Zuchtstämmen von Drosophila melanogaster oder durch Caenorhabditis elegans und deren Enzym Superoxiddismutase gibt, wobei das Enzym entweder in einer besonders aktiven Form oder höherer Quantität vorkommt. Diese Befunde stützen die zur Zeit favorisierte Theorie vom Altern, wonach die permanente Bildung potentiell toxischer, oxidativer Substanzen zu einer Anhäufung irreversibler Schäden (oxidierte Lipide, Proteine und DNS) in Zellen und Geweben führt (8).

Die antiphlogistische Wirkung physiologischer Liposomen, besonders unter Zusatz von Vitamin C, zeigt sich deutlich bei der Behandlung oberflächlicher Brandverletzungen. Werden physiologische Liposomen unmittelbar nach der Hitzeeinwirkung auf das betroffene Hautareal aufgetragen, so kommt es zu einer raschen Schmerzreduktion. Wird die Applikation mehrmals in den darauffolgenden Tagen wiederholt, erfolgt keine Blasenbildung und das unterhalb der zerstörten Haut gelegene Gewebe regeneriert sich wesentlich schneller und (auch bei empfindlichen Hauttypen) ohne Narbenbildung. Ist eine Blasenbildung bereits erfolgt, so führt die Applikation physiologischer Liposomen binnen weniger Stunden zu einem Eintrocknen der Blasen. Somit unterdrücken die Liposomen nicht nur die Extravasation von Plasma ins Gewebe, sondern sorgen auch für eine aktive Rückführung der Flüssigkeit in die Gefäße. Ähnlich restaurierende Effekte wurden nach übermäßiger Einwirkung von UV-Licht beobachtet (Sonnenbrand).

Entzündungen haben viele Gemeinsamkeiten mit allergischen Reaktionen. Da physiologische Liposomen - wie in Abschnitt II dargestellt - eine Unterdrückung der Mediatorfreisetzung und/oder Neutralisierung freigesetzter Mediatoren bewirken, eignen sie sich auch zur Bekämpfung entzündlicher Prozesse. Da Sauerstoffradikale hierbei möglicherweise eine größere Rolle spielen als in der Allergie, ist ein weiterer Zusatz antioxidativer Substanzen, wie z. B. Vitamin C oder Urat, von besonderem Nutzen (auch einige der Liposomenmembran-bildenden Lipide wirken antioxidativ bzw. reduzierend). Im Gegensatz zu der vorher beschriebenen Therapie mit dem Enzym Superoxiddismutase dringen physiologische Liposomen auch in das Gewebe ein und sind zudem voll kompatibel, da es sich bei den membranbildenden Komponenten um Moleküle handelt, wie sie auch im Körper natürlicherweise vorkommen. Bei der intravenösen Applikation können große Mengen physiologischer Liposomen ohne Schaden infundiert werden. Die menschliche Physiologie ist - zeitlich begrenzt - für einen drastischen Anstieg zahlreicher Fettpartikel ausgelegt, wie die große Menge an Chylomikronen im Plasma (Durchmesser 100 - 1000 nm; Bestandteile: 85 % Triglyceride, 5 % Cholesterin, 2 % Protein) nach einem fettreichen Mahl beweist.

Physiologische Liposomen eignen sich zur topischen und systemischen Therapie einer Vielzahl von Erkrankungen des entzündlichen Formenkreises. Neben den bereits Genannten kommen ferner als mögliche Therapieziele in Betracht: Intoxikationen, Schäden bei Beatmung mit hyperbarem Sauerstoff, Arzneimittelnebenwirkungen und Postischämiesyndrom. Bei Endotoxin- bzw. Mikroorganismen-bedingten Schockzuständen und mikrobiell infizierten, größeren Gewebeläsionen dürfte eine gleichzeitige Antibiotikatherapie indiziert sein. Die besonderen antioxidativen Wirkungen sowie die einzigartigen Eindringfähigkeiten in Gewebe lassen physiologische Liposomen auch als Geriatrikum sinnvoll erscheinen.

### Wirkungsmodell physiologischer Liposomen

Die in den vorangegangenen Kapiteln vorgestellten Befunde zeigen, daß physiologische Liposomen sowohl zellulär als auch extrazellulär wirken. Für die zellulären Reaktionen dürften vornehmlich die Auswirkungen der durch die Liposomen auf die Zellmembran übertragenen Gallensäuren und/oder deren Derivate sowie die Lipide verantwortlich sein. Viren werden im extrazellulären Raum durch diese Liposomen inhibiert. Darüberhinaus reagieren liposomale Lipide und liposomal verkapselte Antioxidantien mit Radikalen. Wie man sich diese Wirkungen physiologisch vorstellen kann, wird nachfolgend beschrieben:
**1. Zelluläre Wirkungen physiologischer Liposomen**
   Durch Interaktion von Liposomen mit Zellen werden Gallensäure und/oder ihre Derivate in die Plasmamembran eingebaut.
   1a. Das eingebaute Cholat erhöht infolge herabgesetzter Oberflächenspannung vor allem für Na⁺ (als kleinstes der für das Membranpotential bedeutenden Ionen) die Permeabilität der Membran. Es kommt zu einem geringfügig erhöhten Na⁺-Einstrom in die Zelle. Wasser folgt dem veränderten osmotischen Gradienten und läßt die Zelle solange anschwellen, bis der Innendruck dem Influx das Gleichgewicht halten kann. Zellen innerhalb eines Gewebeverbandes lassen zudem durch die ihnen benachbarten Zellen dieses Anschwellen nur in sehr beschränkten Maße zu, eben nur bis zu derjenigen Größe, welche der Interzellularraum (durchschnittlich 30 nm) erlaubt. Als groben Wert kann man hierfür eine Vergrößerung des Zelldurchmessers um etwa 1 % erwarten. Das Aufquellen der Zellen kann nicht durch die nach Fusion in die Zelle eingebrachten Liposomenvolumina erklärt werden, denn um dieses Zusatzvolumen aufzunehmen, müßte die Zelle die mehr als hundertfache Menge an liposomalem Membranmaterial einbauen, was zellphysiologisch nicht vorstellbar ist. In vivo bedeutet dieses geringfügige Aufquellen, daß sich das Interstitium verkleinert, was wiederum Substanzen (z. B. Mediatoren) und Partikeln (z. B. Viren) eine Ausbreitung erschwert. Den veränderten Ionenkonzentrationen folgend wird vor allem die Na⁺-K⁺-ATPase (Na⁺-Pumpe) unter ATP-Verbrauch versuchen, die ursprünglichen Verhältnisse wiederherzustellen. Unter normalen Bedingungen benötigt dieser Pumpvorgang bereits etwa ein Drittel des gesamten Energiebedarfs einer Zelle (bei elektrisch aktiven Nervenzellen etwa zwei Drittel (12)). Demzufolge dürfte der Energieverbrauch und damit der Gesamtstoffwechsel einer Zelle nach Interaktion mit physiologischen Liposomen ansteigen.
      Entspricht die Interaktion von Liposomen und Zellen einer Fusion, so könnten folgende Ereignisse zutreffen: Nach Fusion der Liposomenmembran mit der Zellmembran wird durch ein ca. 200 nm durchmessendes Liposom ein etwa 0,1 µm² großes Membranstück erhöhter Permeabilität in die Zellmembran (500 µm² Membranaußenfläche bei 12,6 µm Zelldurchmesser) eingebaut. Durch dieses 0,1 µm² große, "neue" Membranstück kommt es zu einem erhöhten Ein- bzw. Ausstrom von Ionen. Da aber Lipide - und vermutlich erst recht Tenside - innerhalb der Lipiddoppelmembran mit einer Geschwindigkeit von 2 µm/sec (7) lateral diffundieren, beginnt bereits zum Zeitpunkt der Fusion das Auseinanderfließen des liposomalen Membranstückes, d. h. es erfolgt eine Vermischung liposomaler und zellulärer Membranbestandteile, die rasch zu einer Abnahme der Ionendurchlässigkeit an dieser Stelle der Zellmembran führt. Bereits eine Ausbreitung der liposomalen Membranbestandteile auf die drei- bis vierfache Fläche (0,3 bis 0,4 µm²) dürfte zu einem wesentlichen Rückgang der Permeabilität führen (abgeleitet aus Experimenten mit Liposomen, die einen niedrigeren Cholatanteil aufweisen). Die hohe Geschwindigkeit lateraler Lipiddiffusion von 2 µm/sec dürfte daher das ursprünglich 0,1 µm² große liposomale Membranstück in Bruchteilen einer Sekunde zum größten Teil "abdichten". Erst wenn eine größere Zahl von Liposomen mit einer Zelle fusioniert, dürften die in die Zellmembran eingebrachten Tenside zu einer die gesamte Zellmembran betreffenden Permeabilitätsänderung führen. In vitro läßt sich zeigen, daß Zellen auf ein kurzzeitiges (15 - 30 min) Angebot sehr hoher Liposomenkonzentrationen negativ reagieren. Nach wenigen Stunden erholen sich die Zellen hiervon jedoch vollständig. Diese Regeneration läßt sich u. a. dadurch erklären, daß 50 % der Plasmamembran pro Stunde (je nach Zelltyp) internalisiert werden, demzufolge die Hälfte der Zellmembran stündlich durch Membranmaterial aus dem Zellinneren ersetzt wird. Der Anteil der Zellmembran am gesamten Membranmaterial einer Zelle macht nur wenige Prozent aus (bei einer Leberzelle ca. 2 %). Die ursprüngliche Permeabilität der Zellmembran wird daher in relativ kurzer Zeit wieder hergestellt.
      Werden den Zellen jedoch hohe Liposomenkonzentrationen auf Dauer angeboten, so können sie sich nicht mehr regenerieren.
   1b. Die nach Liposomenfusion leicht erhöhte Permeabilität der Plasmamembran resultiert in einem geringfügig schwächeren Membranpotential (10). Bei Nervenzellen bzw. deren Ausläufern hat dies zur Folge, daß das Membranpotential näher an die "Zünd-Schwelle" rückt, wodurch nach Impulsgenerierung ein niedrigeres Aktionspotential erzeugt wird. Erste elektrophysiologische Ableitungen an Hippocampusneuronen in vitro zeigen (eigene Resultate), daß in Gegenwart physiologischer Liposomen das Membranpotential (-60 mV) um etwa 4 mV auf -56 mV gebracht wird. Da die Ausschüttung der Neurotransmitter abhängig ist von der Höhe des Aktionspotentials, wird dadurch ein niedrigerer Impuls auf die nächste Zelle (Muskel- oder Nervenzelle) übertragen. Im Falle der Schmerzgenerierung bedeutet dies, daß der Schmerz nachläßt und bei entsprechend erniedrigtem Aktionspotential gänzlich verschwindet. (Ob dem auf physiologische Liposomen ansprechenden tonischen Schmerz bereits ein vom Normalzustand abweichendes Membranpotential der beteiligten Nerven zugrundeliegt und dieses durch Einwirkung der Liposomen erst wieder normalisiert wird, läßt sich zur Zeit nicht belegen.) Falls die Mediatorfreisetzung bei Immunzellen (z. B. Mastzellen) auch vom Membranpotential abhängt und die Granulafreisetzung dieser Zellen der Vesikelfreisetzung an den Synapsen ähnelt, so könnte o. g. Erklärung auch für einige der antiallergischen und antiphlogistischen Eigenschaften physiologischer Liposomen zutreffen.
   1c. Durch die nach Fusion eingebrachten Cholatmoleküle wird die Zellmembran aufgrund herabgesetzter Oberflächenspannung "flüssiger". Auf ihr räumliches Arrangement angewiesene Zelloberflächenstrukturen, wie beispielsweise die "coated pits" oder durch Antigenbindung verursachte IgE-Rezeptoraggregate, könnten durch Cholat "gelockert" werden. Dadurch würde sowohl die Virusbindung als auch die Mastzelldegranulation behindert werden. Auch die komplizierte räumliche Anordnung der Transmernbransegmente des Na⁺-Kanals (7) könnte durch die Cholatmoleküle so verändert werden, daß daraus die unter 1b beschriebenen Wirkungen resultieren. Ferner könnte die räumliche Struktur von Rezeptoren (z. B. für Histamin oder Opiate) sowie deren Zuordnung zu weiteren Membrankomponenten, wie etwa G-Proteine, Adenylatcyclase, Phospholipase C, Proteinkinase C und spannungsabhängigen Ionenkanälen, durch Cholat so beeinflußt werden, daß sich daraus veränderte Werte entsprechender "second messenger" (z. B. cAMP, DAG, IP₃, Ca²⁺) ergeben.
**2. Zelluläre und extrazelluläre Wirkungen physiologischer Liposomen**
   Die in der Liposomenmembran vorhandenen einfach und mehrfach ungesättigten Fettsäuren sowie weitere zusätzlich verkapselte Antioxidantien und Reduktionsmittel helfen durch oxidative Prozesse bereits eingetretene Schäden zu reparieren und die Schäden durch neugebildete Radikale einzudämmen.
   Wie in den Abschnitten I - V beschrieben, zeigt der Zusatz von Vitamin C in vielen Fällen einen verstärkenden Effekt physiologischer Liposomen, obwohl eine reduzierende Wirkung nicht augenfällig erkennbar ist. Dies könnte neben anderen Einflüssen (siehe 10) auch auf folgende zusätzliche Eigenschaft von Vitamin C zurückzuführen sein: Ein nicht unwesentlicher Teil des Vitamin C findet sich innerhalb der liposomalen Lipiddoppelschicht. Hier kann die Ascorbinsäure Einfluß auf die Oberflächenspannung und damit auch auf die Permeabilität der Membran nehmen. So könnte Vitamin C nicht nur durch Reduktion, sondern auch synergistisch zu den Tensiden der Liposomenmembran wirken (wie bei 1a und 1b). Dieser die Permeabilität beeinflussende Effekt könnte auch von anderen Substanzen, wie beispielsweise bestimmten Peptiden, hervorgerufen werden.
**3. Extrazelluläre Wirkungen physiologischer Liposomen**
   Physiologische Liposomen einer bestimmten Konzentration zwingen Viren, mit ihnen zu interagieren, was zur viralen Inaktivierung führt. Die in den Liposomen vorhandenen Tenside dürften dabei maßgeblich zur Inaktivierung der Viren beitragen.

Die folgenden Beispiele und die Figuren erläutern die Erfindung.

### Figurenbeschreibung:

Figur 1A zeigt die Zeitabhängigkeit der Inhibitionsfähigkeit physiologischer Liposomen auf Herpes simplex Typ I-Viren am Beispiel infizierter Verozellen.

Figur 1B ist eine graphische Darstellung der in Fig. 1A gezeigten Ergebnisse.

Figur 2A zeigt die Abhängigkeit der Inhibitionsfähigkeit physiologischer Liposomen von deren Konzentration am Beispiel von mit Herpes simplex Typ I-Viren infizierten Verozellen.

Figur 2B ist eine graphische Darstellung der in Figur 2A gezeigten Ergebnisse.

Figur 3 zeigt die Inhibitionsfähigkeit physiologischer Liposomen in Abhängigkeit von der Konzentration von Herpes simplex Typ I-Viren verschiedener Konzentration.

Figur 4 zeigt den Infekt einer Vorinkubation von Virozellen mit physiologischen Liposornen bei einer nachfolgenden Inkubation mit Herpes simplex Typ I-Viren.

Figur 5 ist eine graphische Darstellung der Größenverteilung der in Beispiel 1 verwendeten Liposomen.

Figur 6 zeigt einen Vergleich der Ohrschmuckverträglichkeit mit und ohne Liposomenbehandlung.

Figur 7 zeigt den Einfluß der Konzentration unterschiedlicher Liposome auf die Kontraktion von isoliertem Meerschweinchendarm.

Figur 8 ist eine graphische Darstellung der in Abhängigkeit von unterschiedlichen Virusdurchmessern erzielbaren dichtesten Packung.

Figur 8A geht von einem Virusdurchrnesser von 120 nm aus; eine Packung wird bei 10,9 x 10¹³ Liposomen/ml erreicht.

Figur 8B geht von einem Virusdurchmesser von 160 nm aus; eine Packung wird bei 6,4 x 10¹³ Liposomen/ml erreicht.

Figur 8C geht von einem Virusdurchmesser von 200 nm aus; eine Packung wird bei 4,2 x 10¹³ Liposomen/ml erreicht.

### Beispiel 1

### Virustatischer Effekt physiologischer Liposomen in vitro am Beispiel des Herpes simplex Typ 1 Virus

### Experiment 1

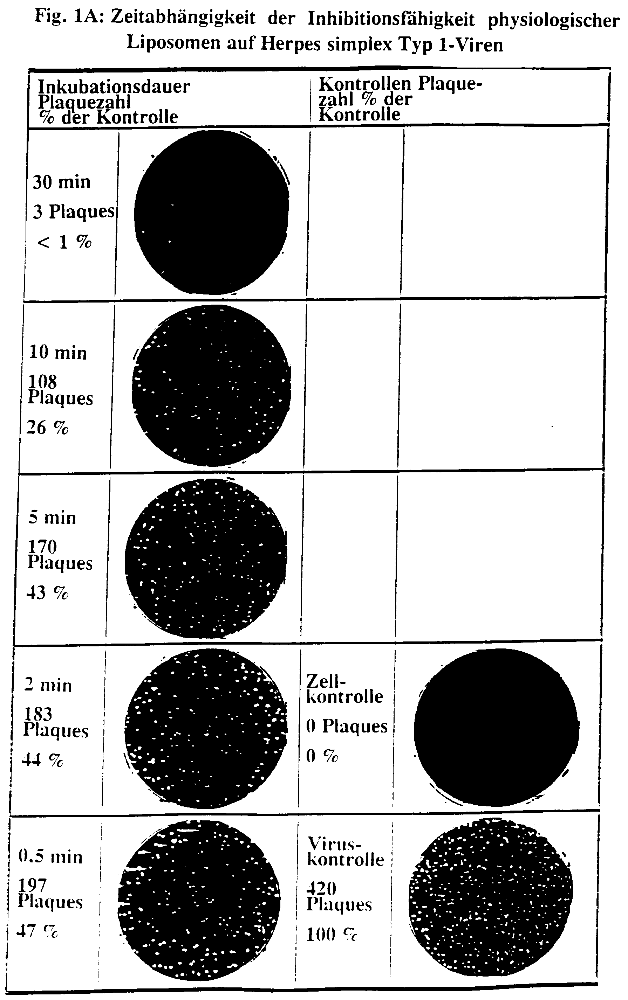

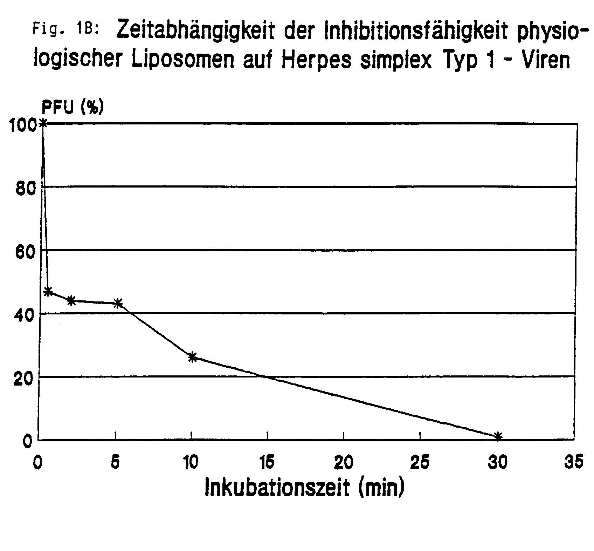

Etwa 420 PFU (plaque forming units) eines frischen Isolats des Herpes simplex Typ 1 (1,2 ml) wurden unterschiedlich lange mit einer Liposomensuspension (1,2 ml) inkubiert, die nach Vermischung 7,3 x 10¹³ Liposomen pro ml - zum allergrößten Teil unilamellare Liposomen - enthielt. Zur Herstellung der Liposomen wurden 3 g Sojalecithin in 3 ml EtOH gelöst. 3 g Natriumascorbat, 0,27 g Kochsalz und 0,4 g Natriurncholat wurden in 23 ml bidest. Wasser gelöst. Beide Lösungen wurden durch Rühren gut vermischt und die heterogene Mischung bei 5 x 10⁶ Pa sterilfiltriert. Die resultierende Liposomendispersion wurde mit 1 N Salzsäure auf pH 6,8 eingestellt und auf die erforderlichen Testkonzentrationen verdünnt. Das Virus-Liposomengemisch wurde dann für 15 Minuten einem konfluenten Monolayer von Affennierenzellen des Typs Vero zugesetzt und anschließend durch Medium ersetzt. Die Auswertung der durch lytische Viren hervorgerufenen PFU erfolgte nach 72 Stunden. Die Ergebnisse dieses Versuchs sind in den Figuren 1a und 1b dargestellt. Für den Zeitpunkt 0 wurde die Kontrolle aufgetragen, d. h. Viren ohne Liposomen den Zellen zugesetzt. Hier wurden 420 PFU nach 3 Tagen gemessen, was in der Abbildung mit 100 % PFU gleichgesetzt wurde. Wurden die Viren mit den Liposomen zusammenpipettiert und sofort anschließend zu den Zellen gegeben, so fanden sich nur mehr 47 % der PFU verglichen mit der Kontrolle. Dieser Zeitraum der Interaktion der Liposomen mit den Viren beträgt etwa 1/2 Minute. Nach 10-minütiger Inkubation der Viren mit den Liposomen verblieben nur noch 26 % PFU, während nach 30 Minuten weniger als 1 % der PFU festgestellt werden konnten. Etwa die Hälfte der Viren wird unmittelbar nach dem Kontakt mit den Liposomen inaktiviert, während einige Viren bis zu 30 Minuten in der Liposomensuspension infektiös bleiben. Dies könnte auf die heterogene Morphologie der Virenhüllen zurückzuführen sein.

### Experiment 2

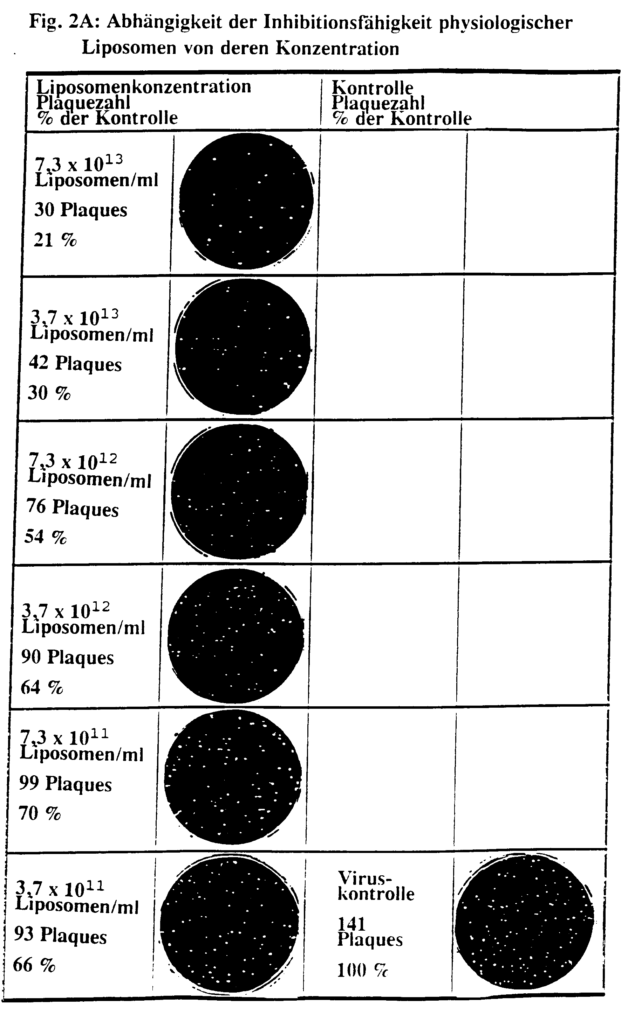

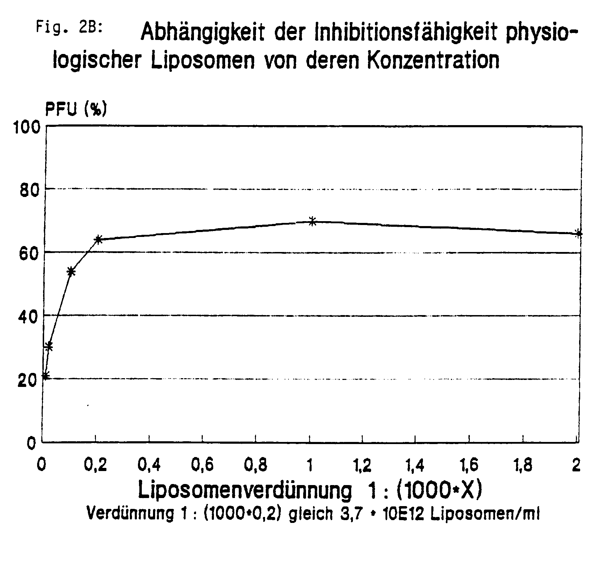

Unterschiedliche Liposomenkonzentrationen (je 1,2 ml) wurden mit der gleichen Anzahl infektiöser Viren (140 PFU in 1,2 ml) für 20 Minuten im Brutschrank inkubiert und dann für 15 Minuten einem Veromonolayer angeboten. Aus dem prozentualen Anteil der PFU, bezogen auf die Kontrolle ohne Liposomenzusatz, ergibt sich die in den Figuren 2a und 2b gezeigte Kinetik. In den ersten Verdünnungsschritten von 7,3 x 10¹³ auf 7,3 x 10¹² Liposomen/ml kommt es zu einem deutlichen Abfall der Inhibierungseffizienz physiologischer Liposomen. Zwischen 3,7 x 10¹² und 3,7 x 10¹¹ Liposomen/ml bleibt die Virusinhibierung konstant bei etwa 30 %. Die rasche Abnahme in der Stärke der Inhibierung bei den ersten Verdünnungsstufen sowie die unveränderte Inhibierungsfähigkeit bei höheren Verdünnungen deuten auf zwei verschiedene Wirkungsmechanismen physiologischer Liposomen hin.

### Experiment 3

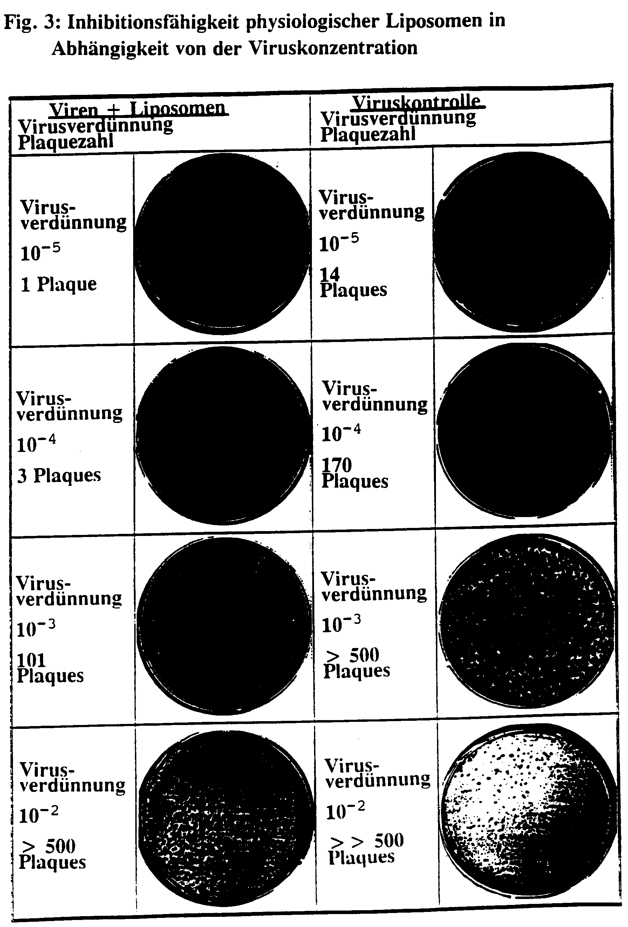

Ein Gemisch aus je einem Volumen (1,2 ml) Liposomensuspension (1,4 x 10¹⁴ Liposomen/ml) und einem Volumen (1,2 ml) einer Virusverdünnungsreihe (10⁻², 10⁻³, 10⁻⁴, 10⁻⁵) wurde hergestellt und im Brutschrank bei 36 C inkubiert. Danach erfolgte das Ausbringen auf Verozellmonolayer: Je 0,5 ml des Gemisches pro Platte wurden für 15 Minuten im Brutschrank adsorbiert, anschließend das Inokulum abgesaugt, einmal mit PBS gewaschen und sodann mit 4 ml Topagar überschichtet. Als Kontrolle diente ein Gemisch von Virus plus PBS. Nach 3 Tagen erfolgte die Auswertung (Figur 3). Bei allen Virusverdünnungsreihen zeigt sich ein deutlicher virustatischer Effekt. In der Verdünnungsstufe 10⁻⁴ beträgt der Unterschied zwischen Liposomen-behandelten Viren (3 PFU) und der Kontrolle (170 PFU) mehr als Faktor 50.

### Experiment 4

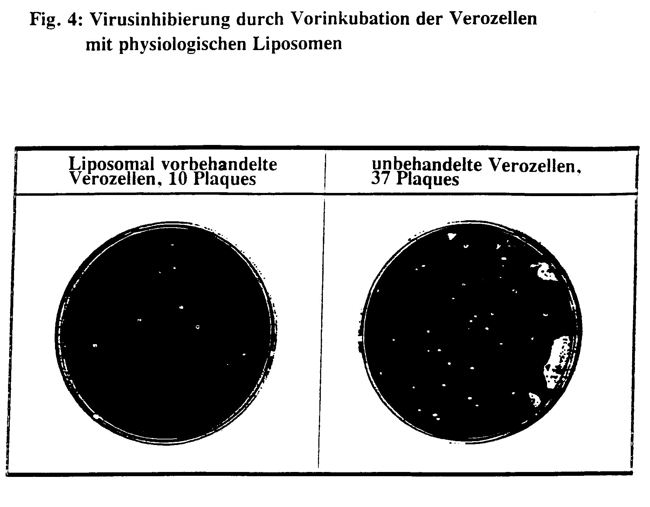

Konfluente Verozellenmonolayer wurden nach Absaugen des Kulturmediums mit PBS gewaschen und mit einer Suspension von 7,3 x 10¹³ Liposomen/ml für 15 Minuten im Brutschrank bei 36 C inkubiert. Danach wurde die Liposomensuspension abgesaugt und die Zellen mit PBS gewaschen. Auf den so vorbehandelten Verozellen wurden Herpesviren in der vorher beschriebenen Weise Plaque-titriert. Als Kontrolle dienten Verozellen, die nicht mit Liposomen vorbehandelt wurden. Das Ergebnis dieses Versuches ist in Figur 4 dargestellt. Auf dem vorbehandelten Verozellmonolayer fanden sich nach 3 Tagen weniger als ein Drittel der Virusplaques verglichen mit der Kontrolle. Daraus läßt sich schließen, daß die Interaktion der Liposomen mit der Zellmembran zu einer gewissen "Immunität" gegenüber dem Eindringen der Herpesviren führt.

### Auswertung des Beispiels 1:

Die aus den Experimenten 1 - 4 erhaltenen Daten weisen auf zwei verschiedene Inhibierungsrnechanismen physiologischer Liposomen hin: Zum einen erfolgt eine Interaktion - vermutlich in Form einer Fusion - der Liposomen mit den Zellen derart, daß ein anschließender Viruskontakt nur noch in einem Drittel der Fälle zu einer erfolgreichen Infektion führt (unter den hier getesteten Bedingungen). Dieser Mechanismus ist wenig abhängig von der Liposomenkonzentration. Zum anderen erfolgt ein virustatischer Effekt durch Wechselwirkung der Liposomen direkt mit den Viren, der stark abhängig ist von der Konzentration der Liposomen.

Ausgehend von der nahezu vollständigen Inhibition der Herpesviren bei einer Liposomenkonzentration von 7,3 x 10¹³/ml (Experiment 1) und der raschen Inhibierungsabnahme bei geringfügig niedrigeren Liposomenkonzentrationen (Experiment 2) ergibt sich ein Zusammenhang aus der Packungsdichte der Liposomen und der Größe der Herpesviren (Durchmesser zwischen 120 und 200 nm), der im Folgenden modellhaft dargestellt ist:

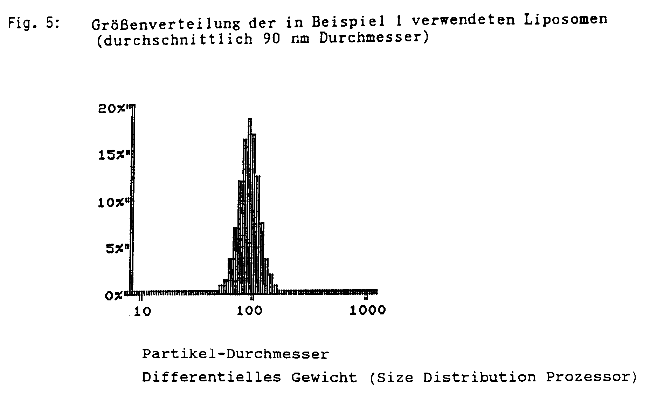

Eine völlige Infektionsinhibierung ergibt sich durch physiologische Liposomen (r = 45 nm) folgender Konzentration (mit Grenzwerten unter Berücksichtigung bi- und multilamellarer Liposomen usw., Abbildung 5):

| | |
|---|---|
| (Minimalkonzentration: | 5,0 x 10¹³/ml) |
| mittlere Konzentration: | 7,3 x 10¹³/ml |
| (Maximalkonzentration: | 12,0 x 10¹³/ml) |

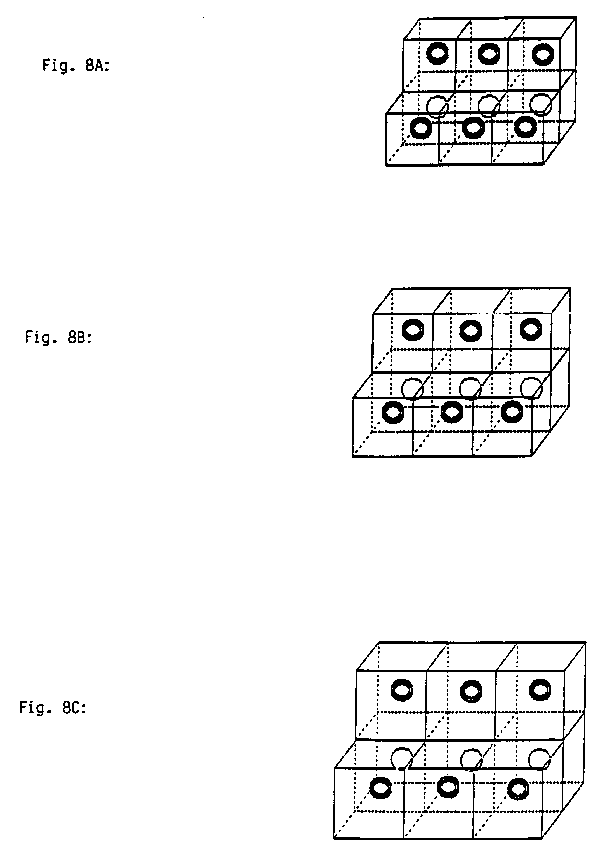

### Minimalwert für kleinste Virusgröße (siehe Fig. 8A)

Herpesvirus r = 60 nm
Minimaler Liposomenabstand: 120 nm
Maximaler Liposomenabstand: 274 nm (räumliche Diagonale zwischen 2 Liposomen)
Seitenlänge Würfel: 210 nm
Volumeninhalt Würfel: 9,2 x 10⁻²¹ m³
Packung wird erreicht bei 10,9 x 10¹³ Liposomen/ml

### Durchschnittliche Virusgröße (siehe Fig. 8B)

Herpesvirus r = 80 nm
Minimaler Liposomenabstand: 160 nm
Maximaler Liposomenabstand: 343 nm
Seitenlänge Würfel: 250 nm
Volumeninhalt Würfel: 1,6 x 10⁻²⁰ m³
Packung wird erreicht bei 6,4 x 10¹³ Liposomen/ml

### Maximalwert für größte Virusgröße (siehe Fig. 8C)

Herpesvirus r = 100 nm
Minimaler Liposomenabstand: 200 nm
Maximaler Liposomenabstand: 412 nm
Seitenlänge Würfel: 290 nm
Volumeninhalt Würfel: 2,4 x 10⁻²⁰ m³
Packung wird erreicht bei 4,2 x 10¹³ Liposomen/ml

Nach den hier vorliegenden Ergebnissen dürfte die effektivste Liposomenkonzentration - je nach Inkubationsdauer und Viruskonzentration - bei etwa 7 x 10¹³ Liposomen/ml liegen. Das Modell berücksichtigt bei den Abstandsberechnungen nicht die Wechselwirkungen in der unmittelbaren Umgebung der Liposomen und Viren (Hydrathüllen, Ionenanlagerung usw.), die möglicherweise zu einer Abstandsvergrößerung und damit zu niedrigerer effektiver Liposomenkonzentration führen. Da ferner die Kräfte unbekannt sind, die eine Interaktion (Fusion) von Liposomen und Herpesviren überhaupt erst ermöglichen, sind über das Modell noch keine genaueren Aussagen machbar. Falls fusogene Proteine bei der Wechselwirkung von Liposomen und Herpesviren keine katalytische Rolle spielen, so kann davon ausgegangen werden, daß sich beide Partikel unter Verdrängung der Wassermoleküle bis auf etwa 1,5 nm annähern müssen, um eine Fusion zu ermöglichen. Es handelt sich dabei um einen energetisch höchst ungünstigen Prozeß (12). Dieser dürfte nur bei einer Liposomendichte stattfinden, wie sie hier vorliegt. Auffällig ist die gute Übereinstimmung zwischen dem theoretischen Durchschnittswert von 6,4 x 10¹³ Liposomen/ml und der nachgewiesenen nahezu 100 % effektiven Liposomenkonzentration von 7,3 x 10¹³/ml sowie die Übereinstimmung der durch das Modell vorherzusagenden raschen Abnahme der Virusinhibierung bei auch geringfügig niedrigeren Liposomenkonzentrationen mit den gemessenen Werten. Bei der topischen Therapie von Herpes-Patienten haben sich Liposomenkonzentrationen um 7 x 10¹³/ml als effektiv herausgestellt. Ein zumindest teilweiser Schutz der Zellen kann auch noch mit niedrigeren Liposomenkonzentrationen erzielt werden, wie im Experiment 2 gezeigt.

### Beispiel 2

### Antiallergische Wirkung physiologischer Liposomen am Beispiel der Ohrschmuckunverträglichkeit

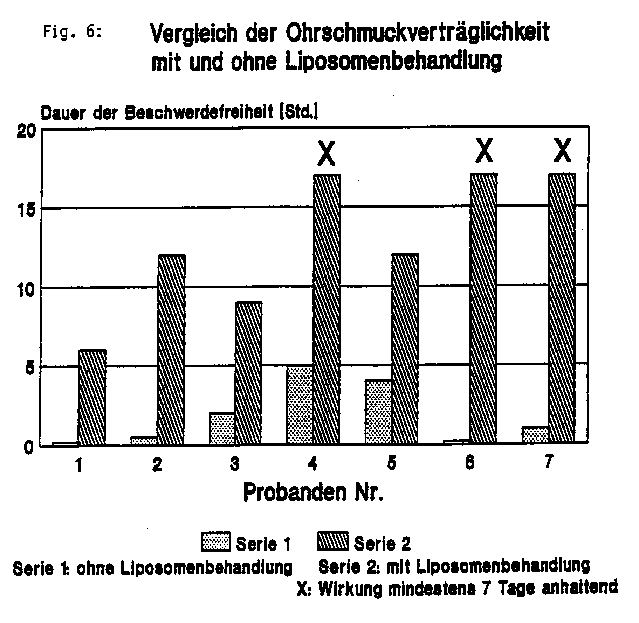

Sieben Patientinnen mit Unverträglichkeit gegenüber Ohrschmuck aus Silber oder unedlen Metallen wurden mit physiologischen Liposomen versorgt. Die Applikation auf das Ohrläppchen erfolgte einmalig oder wiederholt bei Auftreten erneuter Unverträglichkeitsreaktionen. Die Patientinnen wurden befragt, nach welchem Zeitpunkt normalerweise, d. h. ohne liposomale Behandlung, die ersten Symptome einer Unverträglichkeitsreaktion auftraten. Wie aus Abbildung 6 ersichtlich ist, besteht eine breite Varianz in der Reaktion gegenüber unverträglichern Ohrschmuck. Auch sprechen die einzelnen Patienten unterschiedlich stark auf physiologische Liposomen an. Im optimalen Fall tritt nach einmaliger Applikation für mehrere Wochen Beschwerdefreiheit trotz Tragens von sonst unverträglichem Schmuck ein. Aus zahlenmäßig größeren Studien läßt sich ableiten, daß etwa 10 % der Patienten nicht, 20 % nur bedingt und 70 % gut bis sehr gut auf die Behandlung ansprechen.

Vergleichsversuche mit Tween® 20-haltigen Liposomen ergaben keinerlei Linderung der Symptome.

### Beispiel 3

### Nachweis von Membranpotentialveränderungen am isolierten Darm des Meerschweinchens, verursacht durch Liposomen

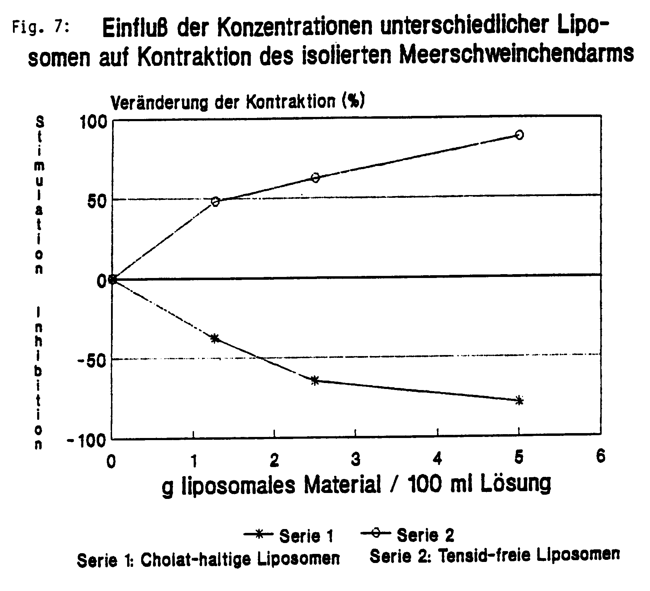

In einem Organbad wurde ein Stück Ileum des Meerschweinchens elektrisch zu Kontraktionen stimuliert, die Stärke der Kontraktionen aufgezeichnet und ausgemessen. Dem Organbad (25 ml Pufferlösung) wurden verschiedene Konzentrationen Cholat-haltiger und Tensid-freier Liposomen (je 0,3 ml) zugesetzt. Die Cholat-haltigen Liposomen wurden hergestellt, wie in Experiment 1 des Beispieles 1 beschrieben, während die Tensid-freien Liposomen durch Beschallung einer Lipid-haltigen, Tensid-freien, wässrigen Lösung erzeugt wurden. Wie aus Abbildung 7 ersichtlich, besteht ein gravierender Unterschied in der Wirkung Cholat-haltiger und Tensid-freier Liposomen. Cholat enthaltende Liposomen (physiologische Liposomen) führen konzentrationsabhängig zu einer Dämpfung der elektrisch stimulierten Darmkontraktionen, während die Tensid-freien Liposomen eine erhebliche Verstärkung der Kontraktion bewirken.

### Beispiel 4

### Physiologische Liposomen zur Behandlung der Dry-Eye Symptomatik

Zwanzig Patienten mit Dry-Eye Symptomatik wurden über einen Zeitraum von 4 Monaten mit physiologischen Liposomen versorgt. Danach erschienen 12 Patienten zur Nachuntersuchung. Die subjektive Beurteilung von Seiten der Patienten ergab:
- wesentliche Besserung:: in 3 Fällen
- wahrnehmbare Besserung:: in 5 Fällen
- keine Besserung:: in 4 Fällen

Bei der objektiven Nachuntersuchung dieser 12 Patienten wurde festgestellt, daß das Spaltlampenbild der Bindehäute in 9 Fällen ruhiger war als vor der Anwendung physiologischer Liposomen. In den 8 Fällen von Patienten, die nicht zur Nachuntersuchung kamen, kann für das Nichterscheinen Beschwerdefreiheit oder therapeutische Resignation angenommen werden.

### Beispiel 5

### Herstellung der erfindungsgemäßen Liposomen

5 g reines Sojalecithin werden in 5 ml Ethanol gelöst. 0,9 g Natriumcholat werden in 85 ml 0,9 %iger Kochsalzlösung gelöst. Beide Lösungen werden bei Raumtemperatur mit 5 x 10⁶ Pa durch einen Filter mit Porengröße 0,22 µm filtriert. Die so erhaltene liposomale Lösung enthält Liposomen mit einem mittleren Durchmesser von 130 nm. Die nach der einmaligen Filtration erhaltene Liposomenlösung kann gegebenenfalls noch auf den gewünschten pH-Wert eingestellt werden.

## Patentansprüche

1. Liposomen ohne darin enthaltenen Wirkstoff zur Verwendung als Arzneimittel, erhältlich durch
(a) Mischen von Doppelschicht-bildenden Lipiden, die mindestens einen Anteil von ungesättigten Fettsäureketten enthalten, wobei die Lipide vor dem Mischen entweder als solche vorliegen oder in einem mit Wasser mischbaren Lösungsmittel gelöst sind und in der Mischung in einer Konzentration von 0,625 mMol/l bis 187,5 mMol/l, bevorzugt 37,7 bis 150 mMol/l und besonders bevorzugt 62,5 bis 125,0 mMol/l vorliegen, mit einer wäßrigen Lösung von Gallensäure und/oder mindestens einem ihrer Derivate, wobei das molare Verhältnis von Lipid zu Gallensäure und/oder deren Derivaten (L:G) 3,1 bis 5,5 beträgt, und
(b) Zuführen mechanischer Energie.

2. Liposomen nach Anspruch 1, dadurch gekennzeichnet, daß die Lipide und/oder die Gallensäure und/oder deren Derivate physiologisch verträgliche Verbindungen sind, bevorzugt natürlich auftretende Verbindungen.

3. Liposomen nach Anspruch 1 oder 2, dadurch gekennzeichnet, daß die Lipide aus der Phospholipide, Sphingolipide und Glykolipide umfassenden Gruppe ausgewählt sind.

4. Liposomen nach einem der Ansprüche 1 bis 3, dadurch gekennzeichnet, daß das mit Wasser mischbare Lösungsmittel mindestens ein Alkohol mit 1 bis 6 Kohlenstoffatomen, bevorzugt Ethanol, ist.

5. Liposomen nach einem der Ansprüche 1 bis 4, dadurch gekennzeichnet, daß der Anteil der ungesättigten Fettsäureketten mindestens 4 Gew.-%, bevorzugt 40 bis 80 Gew.-%, bezogen auf den Gesamtlipidgehalt, beträgt.

6. Liposomen nach einem der Ansprüche 1 bis 5, dadurch gekennzeichnet, daß die Gallensäurederivate aus der Natriumcholat, Natriumdesoxycholat, Natriumglykocholat, Natriumtaurocholat, Natriumtaurodesoxycholat, Natriumursocholat und Natriumchenoxycholat umfassenden Gruppe ausgewählt sind.

7. Liposomen nach einem der Ansprüche 1 bis 6, dadurch gekennzeichnet, daß sie mindestens ein Antioxidans, bevorzugt ein physiologisch verträgliches Antioxidans, enthalten.

8. Liposomen nach Anspruch 7, dadurch gekennzeichnet, daß das Antioxidans aus der BHA, BHT, Octylgallat, Dodecylgallat, Sulfite, Milch-, Zitronen- und Weinsäure und deren Salze, α-Tocopherol, Bilirubin, Vitamin C, Vitamin E, Harnsäure und deren Salze und/oder Derivate davon umfassenden Gruppe ausgewählt sind.

9. Liposomen nach einem der Ansprüche 1 bis 8, dadurch gekennzeichnet, daß sie weiterhin einen oder mehrere übliche Hilfs- und/oder Zusatzstoffe, vorzugsweise Gelbildner, Puffersubstanzen, Membranstabilisatoren und Konservierungsmittel, enthalten.

10. Liposomen nach einem der Ansprüche 1 bis 9, dadurch gekennzeichnet, daß die mechanische Energie zur Herstellung der Liposomen durch einmaliges Filtrieren bei geringem Überdruck, bevorzugt 10⁵ Pa bis 6 x 10⁵ Pa, mit einem Filter einer Porengröße von 0,1 bis 0,8 µm, bevorzugt 0,15 bis 0,3 µm, besonders bevorzugt 0,15 bis 0,22 µm oder durch Rühren, Schütteln oder Homogenisieren zugeführt worden ist.

11. Liposomen nach einem der Ansprüche 1 bis 10 zur Prophylaxe und/oder Therapie von Virusinfektionen bei Mensch, Tier oder Pflanze.

12. Liposomen nach Anspruch 11 zur Prophylaxe und/oder Therapie von Erkrankungen, die von Viren mit Lipidhülle, insbesondere Viren aus der die Familien Herpesviridae, Orthomyxoviridae, Retroviridae und Hepadnaviridae umfassenden Gruppe hervorgerufen werden.

13. Liposomen nach Anspruch 11 zur Prophylaxe und/oder Therapie von Erkrankungen, die von Viren ohne Lipidhülle, insbesondere Viren aus der die Familien Adenoviridae, Papovaviridae und Picornaviridae umfassenden Gruppe, hervorgerufen werden.

14. Liposomen nach einem der Ansprüche 1 bis 10 zur Therapie von durch Mykoplasmataceae, Chlamydiaceae und Rickettsiaceae hervorgerufenen Erkrankungen.

15. Liposomen nach einem der Ansprüche 1 bis 10 zur Prophylaxe und/oder Therapie allergischer Erkrankungen, insbesondere der Haut und der Schleimhaut.

16. Liposomen nach Anspruch 15 zur Prophylaxe und/oder Therapie von Kontakt-, Nahrungsmittel- und Arzneimittelallergien.

17. Liposomen nach Anspruch 15 zur Prophylaxe und/oder Therapie von Allergien des atopischen Formenkreises, insbesondere bei Neurodermitis.

18. Liposomen nach einem der Ansprüche 1 bis 10 zur Prophylaxe und/oder Therapie der trockenen Haut, wobei gegebenenfalls Harnstoff zugesetzt wird.

19. Liposomen nach einem der Ansprüche 1 bis 10 zur Prophylaxe und/oder Therapie von Schmerzen.

20. Liposomen nach Anspruch 19 zur Prophylaxe und/oder Therapie des tonischen Schmerzes.

21. Liposomen nach Anspruch 19 zur Prophylaxe und/oder Therapie von schmerzhaften Muskelverspannungen, Operationsnarben und Phantomschmerz.

22. Liposomen nach einem der Ansprüche 1 bis 10 zur Therapie des Weichteilrheumatismus.

23. Liposomen nach einem der Ansprüche 1 bis 10 zur Behandlung von Zahnhalsempfindlichkeit.

24. Liposomen nach einem der Ansprüche 1 bis 10 zur Prophylaxe und/oder Therapie allergisch und/oder viral bedingter Entzündungen des Auges.

25. Liposomen nach einem der Ansprüche 1 bis 10 zur Prophylaxe und/oder Therapie der Symptomatik des trockenen Auges.

26. Liposomen nach einem der Ansprüche 1 bis 10 zur Prophylaxe und/oder Therapie entzündlicher Erkrankungen.

27. Liposomen nach einem der Ansprüche 1 bis 10 zur Prophylaxe und/oder Therapie von Schädigungen, die durch natürliches oder synthetisches UV-Licht, durch Strahlung aus radioaktivem Zerfall, Röntgenstrahlung oder Hitze verursacht werden.

28. Liposomen nach einem der Ansprüche 1 bis 10 zur Therapie der rheumatoiden Arthritis.

29. Liposomen nach einem der Ansprüche 1 bis 10 zur Prophylaxe und/oder Therapie altersbedingter Beschwerden.

30. Liposomen nach einem der Ansprüche 1 bis 10 als Radikalfänger und/oder zur Soforthilfe bei verschiedenen schockzuständen.

31. Liposomen nach einem der Ansprüche 1 bis 10 zur Behandlung von Erkrankungen, die ganz oder teilweise durch vom Normalzustand abweichende Membranpotentiale der betroffenen Zellen verursacht werden.

32. Liposomen nach einem der Ansprüche 1 bis 10 zur Prophylaxe und/oder Therapie von Erkrankungen, die ganz oder teilweise durch einen vom Normalzustand abweichenden Flüssigkeitsverlust der betroffenen Zellen verursacht werden.

33. Liposomen nach einem der Ansprüche 1 bis 10 zur Prophylaxe und/oder Therapie von Erkrankungen, die ganz oder teilweise durch eine im Verhältnis zum Normalzustand erhöhte Oberflächenspannung der betreffenden Zellmembranen verursacht werden.

34. Liposomen nach einem der Ansprüche 1 bis 10 zur Prophylaxe und/oder Therapie von Erkrankungen, die durch Schädigungen aufgrund oxidativer Prozesse an zellulären und/oder extrazellulären Strukturen hervorgerufen werden.

35. Liposomen nach einem der Ansprüche 1 bis 10 zur Prophylaxe und/oder Therapie von Erkrankungen, die ganz oder teilweise durch vom Normalzustand abweichende intrazelluläre Konzentrationen eines oder mehrerer second messenger, verursacht werden.

36. Verwendung von Liposomen nach einem der Ansprüche 1 bis 35 zur Herstellung eines Arzneimittels.

37. Verwendung nach Anspruch 36 zur Therapie der Neurodermitis, wobei dem Arzneimittel weiterhin ein Lokalanästhetikum in für Hautschutzmittel zulässigen Konzentrationen zugefügt wird, bevorzugt Lidocain oder Tetracain.

38. Verwendung nach Anspruch 36 zur Herstellung eines Hautschutzmittels zur Verwendung im gewerblichen Hautschutz.

39. Arzneimittel, enthaltend Liposomen nach einem der Ansprüche 1 bis 35.

## Claims

1. Liposomes without any active substance contained therein for use as a drug, obtainable by
a) mixing bilayer-forming lipids containing, at least in part, unsaturated fatty-acid chains, wherein prior to mixing the lipids are either present as such or are dissolved in a water-miscible solvent and are present in the mixture in a concentration of from 0.625 mmole/l to 187.5 mmole/l, preferably 37.7 to 150 mmole/l, and 62.5 to 125.0 mmole/l being particularly preferred, with an agueous solution of bile acid and/or at least one derivative thereof, wherein the molar ratio of lipid to bile acid and/or the derivatives thereof (L:G) is 3.1 to 5.5, and
b) supplying mechanical energy.

2. Liposomes according to claim 1, **characterized in** that the lipids and/or the bile acid and/or the derivatives thereof are physiologically tolerated compounds, preferably naturally occurring compounds.

3. Liposomes according to claim 1 or 2, **characterized in** that the lipids are selected from the group consisting of phospholipids, sphingolipids and glycolipids.

4. Liposomes according to any one of claims 1 to 3, **characterized in** that the water-miscible solvent is at least an alcohol having 1 to 6 carbon atoms, preferably ethanol.

5. Liposomes according to any one of claims 1 to 4, **characterized in** that the amount of unsaturated fatty-acid chains is at least 4 wt.%, preferably 40-80 wt.%, based on the total lipid content.

6. Liposomes according to any one of claims 1 to 5, **characterized in** that the bile acid derivatives are selected from the group consisting of sodium cholate, sodium deoxycholate, sodium glycocholate, sodium taurocholate, sodium taurodeoxycholate, sodium ursocholate, and sodium chenoxycholate.

7. Liposomes according to any one of claims 1 to 6, **characterized in** that they contain at least one antioxidant, preferably one physiologically compatible antioxidant.

8. Liposomes according to claim 7, **characterized in** that the antioxidant is selected from the group consisting of BHA, BHT, octyl gallate, dodecyl gallate, sulfites, lactic acid, citric acid and tartaric acid or the salts thereof, α-tocopherol, bilirubin, vitamin C, vitamin E, uric acid and the salts thereof and/or derivatives thereof.

9. Liposomes according to any one of claims 1 to 8, **characterized in** that they further contain one or more conventional adjuvants and/or additives, preferably gel forming agents, buffer substances, membrane stabilizers and/or preservatives.

10. Liposomes according to any one of claims 1 to 9, **characterized in** that the mechanical energy for preparing said liposomes has been supplied by single filtering at a small overpressure, preferably 10⁵ Pa to 6 x 10⁵ Pa, with a filter having a pore size of 0.1 to 0.8 µm, preferably 0.15 to 0.3 µm, 0.15 to 0.22 µm being particularly preferred, or by stirring, shaking or homogenizing.

11. Liposomes according to any one of claims 1 to 10 for the prophylactic and/or therapeutic treatment of virus infections in man, animal or plant.

12. Liposomes according to claim 11 for the prophylactic and/or therapeutic treatment of diseases caused by viruses with lipid envelope, in particular, viruses selected form the group consisting of the families Herpesviridae, Orthomyxoviridae, Retroviridae and Hepadnaviridae.

13. Liposomes according to claim 11 for the prophylactic and/or therapeutic treatment of diseases caused by viruses without lipid envelope, in particular, viruses selected from the group consisting of the families Adenoviridae, Papovaviridae, and Picornaviridae.

14. Liposomes according to any one of claims 1 to 10 for treating diseases caused by Mycoplasmataceae, Chlamydiaceae, and Rickettsiaceae.

15. Liposomes according to any one of claims 1 to 10 for the prophylactic and/or therapeutic treatment of allergic diseases, in particular of the skin and mucous membrane.

16. Liposomes according to claim 15 for the prophylactic and/or therapeutic treatment of contact, foodstuff or drug allergies.

17. Liposomes according to claim 15 for the prophylactic and/or therapeutic treatment of atopic allergies, in particular neurodermatitis.

18. Liposomes according to claims 1 to 10 for the prophylactic and/or therapeutic treatment of dry skin, wherein urea is optionally added.

19. Liposomes according to claims 1 to 10 for the prophylactic and/or therapeutic treatment of pain.

20. Liposomes according to claim 19 for the prophylactic and/or therapeutic treatment of tonic pain.

21. Liposomes according to claim 19 for the prophylactic and/or therapeutic treatment of painful muscle strains, postoperative scars or phantom limb pain.

22. Liposomes according to any one of claims 1 to 10 for the therapeutic treatment of soft-part rheumatism.

23. Liposomes according to any one of claims 1 to 10 for treating dental neck sensitivity.

24. Liposomes according to any one of claims 1 to 10 for the prophylactic and/or therapeutic treatment of allergically and/or virally caused eye inflammations.

25. Liposomes according to any one of claims 1 to 10 for the prophylactic and/or therapeutic treatment of dry-eye symptoms.

26. Lipsomes according to any one of claims 1 to 10 for the prophylactic and/or therapeutic treatment of inflammatory diseases.

27. Liposomes according to any one of claims 1 to 10 for the prophylactic and/or therapeutic treatment of damages caused by natural or synthetic UV light, by radiation from radioactive decay, X-ray radiation or heat.

28. Liposomes according to any one of claims 1 to 10 for the therapeutic treatment of rheumatoid arthritis.

29. Liposomes according to any one of claims 1 to 10 for the prophylactic and/or therapeutic treatment of complaints caused by old age.

30. Liposomes according to any one of claims 1 to 10 as a radical scavenger and/or immediate aid with different states of shock.

31. Liposomes according to any one of claims 1 to 10 for treating diseases which are fully or partly caused by membrane potentials of the affected cells that differ from the normal state.

32. Liposomes according to any one of claims 1 to 10 for the prophylactic and/or therapeutic treatment of diseases which are fully or partly caused by a fluid loss of the affected cells that differs from the normal state.

33. Liposomes according to any one of claims 1 to 10 for the prophylactic and/or therapeutic treatment of diseases which are fully or partly caused by a surface tension of the affected cell membranes which is increased relative to the normal state.

34. Liposomes according to any one of claims 1 to 10 for the prophylactic and/or therapeutic treatment of diseases which are caused by damage due to oxidative processes on cellular and/or extracellular structures.

35. Liposomes according to any one of claims 1 to 10 for the prophylactic and/or therapeutic treatment of diseases which are fully or partly caused by intracellular concentrations of one or several second messengers which differ from the normal state.

36. Use of liposomes according to any one of claims 1 to 35 for the preparation of a drug.

37. Use according to claim 36 for treating neurodermatitis, wherein the drug has further added a local anesthetic in concentrations allowed for skin protectives, preferably lidocaine or tetracaine.

38. Use according to claim 36 for preparing a skin protective suited for use in commercial skin protection.

39. A drug containing liposomes according to any one of claims 1 to 35.

## Revendications

1. Liposomes ne contenant pas de principe actif destinés à être utilisés comme médicament, pouvant être obtenus en
(a) mélangeant des lipides formant des doubles couches et contenant au moins une partie de chaînes d'acides gras insaturés, les lipides étant présents avant mélange soit en tant que tels, soit en solution dans un solvant miscible à l'eau, et à une concentration dans le mélange de 0,625 mmol/l à 187,5 mmol/l, de préférence de 37,7 à 150 mmol/l, et de façon préférée de 62,5 à 125,0 mmol/l, avec une solution aqueuse d'acide cholique et/ou d'au moins un de ses dérivés, le rapport molaire du lipide sur l'acide cholique et/ou les dérivés de celui-ci (L:Ch) étant de 3,1 à 5,5, et
(b) en apportant de l'énergie mécanique.

2. Liposomes selon la revendication 1, caractérisés en ce que les lipides et/ou l'acide cholique et/ou les dérivés de ce dernier sont des composés de liaisons physiologiquement compatibles, de préférence de liaisons naturellement présentes.

3. Liposomes selon la revendication 1 ou la revendication 2, caractérisés en ce que les lipides sont choisis dans le groupe comprenant les phospholipides, les sphingolipides et les glycolipides.

4. Liposomes selon l'une quelconque des revendications 1 à 3, caractérisés en ce que le solvant miscible à l'eau est un alcool possédant de 1 à 6 atomes de carbone, de préférence l'éthanol.

5. Liposomes selon l'une quelconque des revendications 1 à 4, caractérisés en ce que la proportion des chaînes d'acides gras insaturés est au moins égale à 4% en poids, de préférence de 40 à 80% en poids, ramenée à la teneur totale en lipides.

6. Liposomes selon l'une quelconque des revendications 1 à 5, caractérisés en ce que les dérivés de l'acide cholique sont choisis dans le groupe comprenant le cholate de sodium, le désoxycholate de sodium, le glycocholate de sodium, le taurocholate de sodium, le taurodésoxycholate de sodium, l'ursocholate de sodium et le chénoxycholate de sodium.

7. Liposomes selon l'une quelconque des revendications 1 à 6, caractérisés en ce qu'ils contiennent au moins un antioxydant, de préférence un antioxydant physiologiquement compatible.

8. Liposomes selon la revendication 7, caractérisés en ce que l'antioxydant est choisi dans le groupe comprenant le BHA, le BHT, le gallate d'octyle, le gallate de dodécyle, des sulfites, les acides lactique, citrique et tartrique ainsi que leurs sels, l'alphatocophérol, la bilirubine, la vitamine C, la vitamine E, l'acide urique et ses sels, et/ou des dérivés de ces substances.

9. Liposomes selon l'une quelconque des revendications 1 à 8, caractérisés en ce qu'ils contiennent en outre un ou plusieurs adjuvants et/ou additifs habituels, de préférence des agents gélifiants, des substances tampons, des stabilisateurs de membrane et des milieux conservateurs.

10. Liposomes selon l'une quelconque des revendications 1 à 9, caractérisés en ce que l'énergie mécanique pour la fabrication des liposomes a été fournie par une filtration unique sous une faible surpression, de préférence de 10⁵ Pa à 6 x 10⁵ Pa, au moyen d'un filtre ayant une taille de pores de 0,1 à 0,8 µm, de préférence de 0,15 à 0,3 µm, et de façon particulièrement préférée de 0,15 à 0,22 µm, ou bien par agitation, secouage ou homogénéisation.

11. Liposomes selon l'une quelconque des revendications 1 à 10 destinés à la prophylaxie et/ou au traitement d'infections virales chez l'homme, l'animal ou les végétaux.

12. Liposomes selon la revendication 11 destinés à la prophylaxie et/ou au traitement d'affections dues à des virus à enveloppe lipidique, notamment des virus du groupe comprenant les familles des herpèsviridés, orthomyxoviridés, rétroviridés et hépadnaviridés.

13. Liposomes selon la revendication 11 destinés à la prophylaxie et/ou au traitement d'affections dues à des virus sans enveloppe lipidique, notamment des virus du groupe des comprenant les familles des adénoviridés, papovaviridés et picornaviridés.

14. Liposomes selon l'une quelconque des revendications 1 à 10 destinées au traitement d'affections dues à des mycoplasmatacées, des chlamydiacées et des rickettsiacées.

15. Liposomes selon l'une quelconque des revendications 1 à 10 destinés à la prophylaxie et/ou au traitement d'affections allergiques, notamment de la peau et des muqueuses.

16. Liposomes selon la revendication 15, destinés à la prophylaxie et/ou au traitement d'allergies de contact, alimentaires et médicamenteuses.

17. Liposomes selon la revendication 15 destinés à la prophylaxie et/ou au traitement d'allergies de type atopique, notamment la névrodermite.

18. Liposomes selon l'une quelconque des revendications 1 à 10 destinés à la prophylaxie et/ou au traitement de la peau sèche, avec le cas échéant addition d'urée.

19. Liposomes selon l'une quelconque des revendications 1 à 10 destinés à la prophylaxie et/ou au traitement de la douleur.

20. Liposomes selon la revendication 19 destinés à la prophylaxie et/ou au traitement de la douleur tonique.

21. Liposomes selon la revendication 19 destinés à la prophylaxie et/ou au traitement des contractures musculaires douloureuses, des cicatrices postopératoires et des pseudodouleurs.

22. Liposomes selon l'une quelconque des revendications 1 à 10 destinés au traitement du rhumatisme des parties molles.

23. Liposomes selon l'une quelconque des revendications 1 à 10 destinés au traitement de la sensibilité du collet de la dent.

24. Liposomes selon l'une quelconque des revendications 1 à 10 destinés à la prophylaxie et/ou au traitement d'inflammations oculaires d'origine allergique et/ou virale.

25. Liposomes selon l'une quelconque des revendications 1 à 10 destinés à la prophylaxie et/ou au traitement du syndrome de l'oeil sec.

26. Liposomes selon l'une quelconque des revendications 1 à 10 destinés à la prophylaxie et/ou au traitement des affections inflammatoires.

27. Liposomes selon l'une quelconque des revendications 1 à 10 destinés à la prophylaxie et/ou au traitement de lésions dues à la lumière ultraviolette naturelle ou artificielle, au rayonnement provenant d'une désintégration radioactive, à une exposition aux rayons X ou à la chaleur.

28. Liposomes selon l'une quelconque des revendications 1 à 10 destinés au traitement de la polyarthrite rhumatoïde.

29. Liposomes selon l'une quelconque des revendications 1 à 10 destinés à la prophylaxie et/ou au traitement des troubles liés à l'âge.

30. Liposomes selon l'une quelconque des revendications 1 à 10 en tant qu'agents de capture de radicaux et/ou destinés aux premiers secours dans divers états de choc.

31. Liposomes selon l'une quelconque des revendications 1 à 10 destinés au traitement d'affections dues en tout ou partie à un potentiel membranaire des cellules concernées s'écartant de la normale.

32. Liposomes selon l'une quelconque des revendications 1 à 10 destinés à la prophylaxie et/ou au traitement d'affections dues en tout ou partie à une perte de liquide des cellules concernées s'écartant de la normale.

33. Liposomes selon l'une quelconque des revendications 1 à 10 destinés à la prophylaxie et/ou au traitement d'affections dues en tout ou partie à une tension de surface des membranes cellulaires concernées s'écartant de la normale.

34. Liposomes selon l'une quelconque des revendications 1 à 10 destinés à la prophylaxie et/ou au traitement d'affections dues à des lésions consécutives à des processus d'oxydation des structures cellulaires et/ou extracellulaires.

35. Liposomes selon l'une quelconque des revendications 1 à 10 destinés à la prophylaxie et/ou au traitement d'affections dues en tout ou partie à une concentration intracellulaire d'un ou plusieurs second(s) messager(s) s'écartant de la normale.

36. Utilisation des liposomes selon l'une quelconque des revendications 1 à 35 pour la fabrication d'un médicament.

37. Utilisation selon la revendication 36 pour le traitement de la névrodermite, dans laquelle un anesthésique local est ajouté, en plus au médicament, à des concentrations admissibles pour les agents dermoprotecteurs, de préférence de la lidocaïne ou de la tétracaïne.

38. Utilisation selon la revendication 36 pour la fabrication d'un agent dermoprotecteur destiné à la protection de la peau en milieu professionnel.

39. Médicament contenant des liposomes selon l'une quelconque des revendications 1 à 35.
